(19) Europäisches Patentamt
European Patent Office
Office européen des brevets

(11) EP 2 194 082 B1

(12) **EUROPÄISCHE PATENTSCHRIFT**

(45) Veröffentlichungstag und Bekanntmachung des Hinweises auf die Patenterteilung:
**07.09.2011 Patentblatt 2011/36**

(51) Int Cl.:
***C08G 18/12*** *(2006.01)* ***C09J 175/00*** *(2006.01)*
***C07C 251/08*** *(2006.01)*

(21) Anmeldenummer: **08170476.9**

(22) Anmeldetag: **02.12.2008**

(54) **Aldimin und Aldimin enthaltende Zusammensetzung**

Aldimine and composition comprising aldimine

Aldimine et composition contenant aldimine

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(43) Veröffentlichungstag der Anmeldung:
**09.06.2010 Patentblatt 2010/23**

(73) Patentinhaber: **Sika Technology AG**
**6340 Baar (CH)**

(72) Erfinder: **Burckhardt, Urs**
**8049 Zürich (CH)**

(74) Vertreter: **Sika Patent Attorneys**
**C/o Sika Technology AG**
**Tüffenwies 16**
**Postfach**
**8048 Zürich (CH)**

(56) Entgegenhaltungen:
**EP-A- 0 947 529** **EP-A- 1 975 190**
**WO-A-2007/036571** **DE-A1- 1 670 133**
**DE-A1- 2 546 536**

- **MÖHRLE H ET AL: "Die Aminomethylierung von Isobutyraldehyd mit freien primären Aminen in homogenem und heterogenem Medium. Über Mannichbasen XI" DIE PHARMAZIE NOV 1975, Bd. 30, Nr. 11, November 1975 (1975-11), Seiten 699-706, XP009114709 ISSN: 0031-7144**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

### Technisches Gebiet

Die Erfindung betrifft das Gebiet der Aldimine und das Gebiet der Polyurethanzusammensetzungen sowie deren Verwendung, insbesondere als Klebstoff, Dichtstoff, Beschichtung oder Bodenbelag.

### Stand der Technik

Aldimine sind Kondensationsprodukte aus primären Aminen und Aldehyden und stellen eine seit langem bekannte Stoffklasse dar. Bei Kontakt mit Feuchtigkeit können Aldimine zu den entsprechenden Aminen und Aldehyden hydrolysieren. Aufgrund dieser Eigenart können sie als geschützte Form von Aminen, beziehungsweise von Aldehyden, verwendet werden. So werden Aldimine beispielsweise in der Polyurethanchemie eingesetzt, wo sie als durch Feuchtigkeit aktivierbare Vernetzer, sogenannte "blockierte Amine" oder "latente Härter", für ein- oder zweikomponentige Isocyanatgruppen aufweisende Zusammensetzungen dienen.

Die Vorteile der Verwendung von Aldiminen als latente Härter in Isocyanatgruppen aufweisenden Systemen liegen insbesondere darin, dass sich die Entstehung von unerwünschten Gasblasen vermeiden lässt, da die Aushärtungsreaktion über das blockierte Amin - im Gegensatz zur direkten Reaktion von Isocyanaten mit Feuchtigkeit - nicht unter Freisetzung von Kohlendioxid ($CO_2$) verläuft, und dass sich höhere Aushärtungsgeschwindigkeiten und/oder längere Offenzeiten erzielen lassen. Der Einsatz von Aldiminen als latente Härter in Isocyanatgruppen aufweisenden Zusammensetzungen kann aber auch Probleme verursachen. Bei einkomponentigen Zusammensetzungen kann die Lagerstabilität durch die Anwesenheit des Aldimins stark eingeschränkt sein. In Abhängigkeit der zur Herstellung des Aldimins verwendeten und bei der Aushärtungsreaktion wieder freigesetzten Aldehyde können die Zusammensetzungen zudem einen sehr starken Geruch aufweisen, welcher für viele Anwendungen nicht tolerierbar ist.

WO 2004/013088 A1 beschreibt geruchsfreie Polyaldimine, welche aus primären Polyaminen und geruchsfreien Aldehyden hergestellt werden. WO 2007/036571 A1 beschreibt geruchsfreie Aldimine enthaltend mindestens eine Hydroxyl-, Mercapto- oder sekundäre Aminogruppe, welche ebenfalls ausgehend von geruchsfreien Aldehyden erhältlich sind. Diese geruchsfreien Aldehyde können in Polymerzusammensetzungen, insbesondere in Polyurethanzusammensetzungen, eine stark weichmachende Wirkung haben, was unerwünscht sein kann. Das relativ hohe Molekulargewicht der Aldehyde führt überdies dazu, dass die daraus hergestellten Aldimine als latente Härter in relativ grosser Menge eingesetzt werden müssen, was ihren Einsatz teuer machen kann.

### Darstellung der Erfindung

Aufgabe der vorliegenden Erfindung ist es daher, neue Aldimine zur Verfügung zu stellen, welche als latente Härter in härtbaren Zusammensetzungen, insbesondere in feuchtigkeitshärtenden, Isocyanatgruppen aufweisenden Polyurethanzusammensetzungen, eingesetzt werden können.

Überraschenderweise wurde gefunden, dass Aldimine nach Anspruch 1 diese Aufgabe lösen und vorteilhafte Eigenschaften aufweisen. Es handelt sich dabei um bei Raumtemperatur meist flüssige Verbindungen, welche vor, während und nach der Hydrolyse kaum Aldehyd-Geruch aufweisen. Sie weisen eine gute Lagerstablität zusammen mit Isocyanatgruppen auf, insbesondere auch mit sehr reaktiven aromatischen Isocyanatgruppen. Bei ihrer Hydrolyse wird ein Aldehyd freigesetzt, welcher in Polyurethanzusammensetzungen besonders gut verträglich ist, dort nur eine geringe weichmachende Wirkung ausübt und kaum zu Migration oder Ausschwitzen neigt. Sie sind deshalb sehr gut geeignet als latente Härter in Isocyanatgruppen aufweisenden Zusammensetzungen.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Ein Gegenstand der Erfindung ist ein Aldimin der Formel (I),

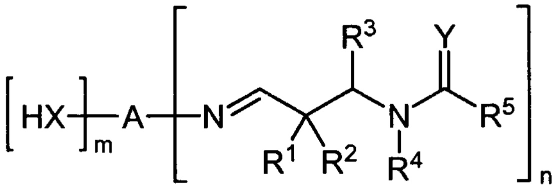

(I)

wobei

Y für O oder S steht;
A entweder für den Rest eines Amins nach Entfernung von n primären Aminogruppen und m HX-Gruppen steht, oder zusammen mit $R^7$ für einen (n+2*m)-wertigen Kohlenwasserstoffrest mit 3 bis 20 C-Atomen, welcher gegebenenfalls mindestens ein Heteroatom, insbesondere in der Form von Ether-Sauerstoff oder tertiärem Amin-Stickstoff, enthält, steht;
n für 1 oder 2 oder 3 oder 4 steht, und
m für 0 oder 1 oder 2 oder 3 oder 4 steht,
mit der Massgabe, dass m+n für 2 oder 3 oder 4 oder 5 steht;
$R^1$ und $R^2$ entweder

unabhängig voneinander jeweils für einen einwertigen Kohlenwasserstoffrest mit 1 bis 12 C-Atomen stehen, oder zusammen für einen zweiwertigen Kohlenwasserstoffrest mit 4 bis 12 C-Atomen, der Teil eines, gegebenenfalls substituierten, carbocyclischen Rings mit 5 bis 8, bevorzugt 6, C-Atomen ist, stehen;

$R^3$ für ein Wasserstoffatom oder einen Alkyl-, Cycloalkyl-, Arylalkyl- oder Alkoxycarbonylrest mit 1 bis 12 C-Atomen steht;
$R^4$ und $R^5$ entweder
zusammen für einen gegebenenfalls Sauerstoff- oder Schwefelatome aufweisenden zweiwertigen Rest mit 2 bis 10 C-Atomen, der Teil eines, gegebenenfalls substituierten, 5- oder 6- oder 7-gliedrigen Rings ist, stehen, oder
$R^4$ für einen Alkyl-, Cycloalkyl-, Arylalkyl- oder Acylrest mit 1 bis 10 C-Atomen steht, und
$R^5$ für ein Wasserstoffatom oder für einen einwertigen Rest mit 1 bis 20 C-Atomen ausgewählt aus der Gruppe bestehend aus Alkyl-, Cycloalkyl-, Arylalkyl-, Arylrest, -$OR^{5'}$, -$SR^{5'}$ und -$NR^{5'}R^{5''}$ steht, wobei $R^{5'}$ und $R^{5''}$ entweder jeweils für einen Kohlenwasserstoffrest oder zusammen für einen Alkylenrest, der Teil eines 5-, 6- oder 7-gliedrigen Rings ist, stehen;
X für O oder S oder N-$R^6$ oder N-$R^7$ steht,

wobei $R^6$
entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
oder für einen Substituenten der Formel (II) steht,

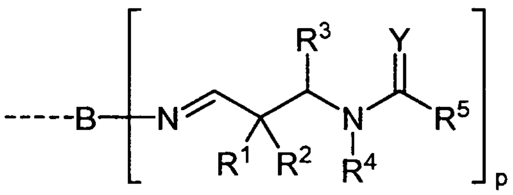

(II)

wobei

p für 0 oder für eine ganze Zahl von 1 bis 10'000 steht, und
B für einen (p+1)-wertigen Kohlenwasserstoffrest, welcher gegebenenfalls Ether-Sauerstoff, tertiären Amin-Stickstoff, Hydroxylgruppen, sekundäre Aminogruppen oder Mercaptogruppen enthält, steht; und

$R^7$ zusammen mit A für einen (n+2*m)-wertigen Kohlenwasserstoffrest mit 3 bis 20 C-Atomen, welcher gegebenenfalls mindestens ein Heteroatom, insbesondere in Form von Ether-Sauerstoff oder tertiärem Amin-Stickstoff, enthält, steht.

Die gestrichelten Linien in den Formeln in diesem Dokument stellen jeweils die Bindung zwischen einem Substituenten und dem zugehörigen Molekülrest dar.

Der Begriff "primäre Aminogruppe" bezeichnet im vorliegenden Dokument eine Aminogruppe in der Form einer $NH_2$-Gruppe, die an einen organischen Rest gebunden ist. Der Begriff "sekundäre Aminogruppe" bezeichnet eine Aminogruppe, in der das Stickstoffatom an zwei organische Reste gebunden ist, welche auch gemeinsam Teil eines Rings

sein können. Der Begriff "tertiäre Aminogruppe" bezeichnet eine Aminogruppe, in der das Stickstoffatom (= tertiärer Amin-Stickstoff) an drei organische Reste gebunden ist, wobei zwei dieser Reste auch gemeinsam Teil eines Rings sein können.

Der Begriff "aktiver Wasserstoff" bezeichnet im vorliegenden Dokument das Wasserstoffatom einer Hydroxyl-, einer Mercapto- oder einer sekundären oder primären Aminogruppe.

Als "aliphatisch" wird ein Amin und ein Isocyanat bezeichnet, deren Amino- und Isocyanatgruppen jeweils ausschliesslich an aliphatische, cycloaliphatische oder arylaliphatische Reste gebunden sind; entsprechend werden diese Gruppen als aliphatische Amino- und Isocyanatgruppen bezeichnet.

Als "aromatisch" wird ein Amin und ein Isocyanat bezeichnet, deren Amino- und Isocyanatgruppen jeweils an einen aromatischen Rest gebunden sind; entsprechend werden diese Gruppen als aromatische Amino- und Isocyanatgruppen bezeichnet.

Unter einer "geruchsarmen" Substanz wird eine Substanz verstanden, deren Geruch von menschlichen Individuen nur in geringem Masse wahrnehmbar, d.h. riechbar, ist, die also keinen intensiven Geruch aufweist wie beispielsweise Formaldehyd, Acetaldehyd, Isobutyraldehyd, oder Lösemittel wie Aceton, Methylethylketon oder Methylisobutylketon, und wobei dieser geringe Geruch von den meisten menschlichen Individuen nicht als unangenehm oder abstossend empfunden wird.

Unter einer "geruchsfreien" Substanz wird eine Substanz verstanden, die für die meisten menschlichen Individuen nicht riechbar ist, die also keinen wahrnehmbaren Geruch aufweist.

Bevorzugt stehen $R^1$ und $R^2$ jeweils für einen Methylrest.

Bevorzugt steht $R^3$ für ein Wasserstoffatom.

Bevorzugt steht Y für ein Sauerstoffatom.

Bevorzugt steht $R^4$ für einen Methyl-, Ethyl-, Propyl-, Isopropyl-, Butyl-, 2-Ethylhexyl-, Cyclohexyl- oder Benzylrest und $R^5$ für ein Wasserstoffatom oder einen Methyl-, Ethyl-, Propyl-, Isopropyl-, Butyl-, 2-Ethylhexyl-, Cyclohexyl-, Benzyl-, Methoxy-, Ethoxy-, Propoxy- oder Isopropoxyrest, oder $R^4$ und $R^5$ bilden zusammen - unter Einbezug des Stickstoffatoms und der Carbonyl- bzw. Thiocarbonylgruppe - einen Ring, insbesondere einen 2-Pyrrolidon-Ring, einen Pyrrolidin-2,5-dion-Ring, einen Piperidin-2-on-Ring, einen Piperidin-2,6-dion-Ring, einen Azepan-2-on-Ring, einen Oxazolidin-2-on-Ring oder einen Thiazolidin-2-on-Ring, wobei ein solcher Ring gegebenenfalls substituiert ist.

In einer Ausführungsform der Aldimine der Formel (I) steht m für 1 oder 2 oder 3 oder 4, bevorzugt für 1. Solche Aldimine weisen also - herrührend von der HX-Gruppe - mindestens einen aktiven Wasserstoff auf.

Besonders bevorzugte Aldimine der Formel (I) mit mindestens einem aktiven Wasserstoff stellen Aldimine der Formel (I a) dar,

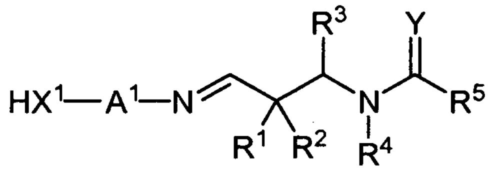

(I a)

wobei

$A^1$ entweder

für einen zweiwertigen Kohlenwasserstoffrest mit 2 bis 20 C-Atomen, welcher gegebenenfalls mindestens ein Heteroatom, insbesondere in der Form von Ether-Sauerstoff oder tertiärem Amin-Stickstoff, enthält, steht,

oder

zusammen mit $R^9$ für einen dreiwertigen Kohlenwasserstoffrest mit 3 bis 20 C-Atomen, welcher gegebenenfalls mindestens ein Heteroatom, insbesondere in der Form von Ether-Sauerstoff oder tertiärem Amin-Stickstoff, enthält, steht;

$X^1$ für O oder S oder N-$R^8$ oder N-$R^9$ steht,
wobei $R^8$
entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht, oder für einen Substituenten der Formel (II a) steht,

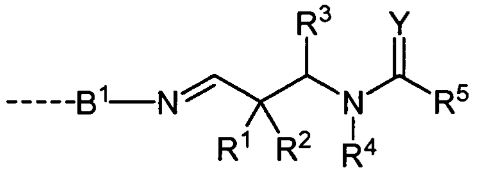

(II a)

wobei $B^1$ für einen zweiwertigen, gegebenenfalls Ether-Sauerstoff oder tertiären Amin-Stickstoff aufweisenden, Kohlenwasserstoffrest mit 2 bis 12 C-Atomen steht; und

$R^9$ zusammen mit $A^1$ für einen dreiwertigen Kohlenwasserstoffrest mit 3 bis 20 C-Atomen, welcher gegebenenfalls mindestens ein Heteroatom,
insbesondere in der Form von Ether-Sauerstoff oder tertiärem Amin-Stickstoff, enthält, steht,
und Y, $R^1$, $R^2$, $R^3$, $R^4$ und $R^5$ die bereits genannten Bedeutungen aufweisen, mit der Massgabe, dass $A^1$ keinen aktiven Wasserstoff aufweist.

In einer weiteren Ausführungsform der Aldimine der Formel (I) steht m für null und n für 2 oder 3 oder 4. Solche Aldimine stellen Polyaldimine dar. Mit "Poly" beginnende Substanznamen wie Polyaldimin, Polyamin oder Polyisocyanat bezeichnen im vorliegenden Dokument Substanzen, die formal zwei oder mehr der in ihrem Namen vorkommenden funktionellen Gruppen pro Molekül enthalten.
Besonders bevorzugte Aldimine der Formel (I) mit m = 0 stellen Aidimine der Formel (Ib) dar,

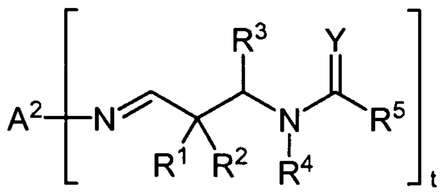

(I b)

wobei

t für 2 oder 3 steht;
$A^2$ für den Rest eines Amins **B2** nach Entfernung von t primären Aminogruppen steht,
und Y, $R^1$, $R^2$, $R^3$, $R^4$ und $R^5$ die bereits genannten Bedeutungen aufweisen, mit der Massgabe, dass das Aldimin der Formel (I b) keinen aktiven Wasserstoff aufweist.

Aldimine der Formel (I) sind erhältlich aus der Umsetzung von mindestens einem Amin **B** der Formel (III) mit mindestens einem Aldehyd **ALD** der Formel (IV),

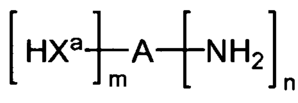

(III)

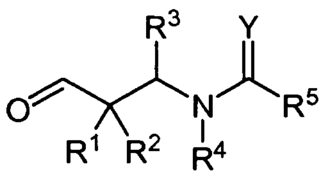

(IV)

wobei

$X^a$ für O oder S oder N-$R^{6a}$ oder N-$R^7$ steht,
wobei $R^{6a}$ entweder für einen einwertigen Kohlenwasserstoffrest mit 1 bis 20 C-Atomen, welcher gegebenenfalls

mindestens eine Carbonsäureester-, Nitril-, Nitro-, Phosphonsäureester-, Sulfon- oder Sulfonsäureester-Gruppe aufweist, steht,
oder für einen Substituenten der Formel (III') steht,

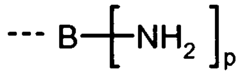

(III')

und m, n, A, B, Y, $R^1$, $R^2$, $R^3$, $R^4$, $R^5$ und p die bereits genannten Bedeutungen aufweisen.

Die Umsetzung zwischen einem Amin **B** der Formel (III) und einem Aldehyd **ALD** der Formel (IV) erfolgt in einer Kondensationsreaktion unter Abspaltung von Wasser. Solche Kondensationsreaktionen sind bestens bekannt und beschrieben, beispielsweise in Houben-Weyl, "Methoden der organischen Chemie", Vol. XI/2, Seite 73ff. Der Aldehyd **ALD** wird hierbei in Bezug auf die primären Aminogruppen des Amins **B** stöchiometrisch oder in stöchiometrischem Überschuss eingesetzt. Üblicherweise werden solche Kondensationsreaktionen in Anwesenheit eines Lösemittels durchgeführt, mittels welchem das bei der Reaktion entstehende Wasser azeotrop entfernt wird. Zur Herstellung der Aldimine der Formel (I) wird jedoch ein Herstellverfahren ohne Verwendung von Lösemitteln bevorzugt, wobei das bei der Kondensation gebildete Wasser direkt mittels Anlegen von Vakuum aus der Reaktionsmischung entfernt wird. Durch die lösemittelfreie Herstellung erübrigt sich ein Abdestillieren des Lösemittels nach erfolgter Herstellung, was den Herstellungsprozess vereinfacht. Zudem ist das Aldimin so frei von Lösemittelrückständen, welche einen störenden Geruch verursachen könnten.

Als Amin **B** der Formel (III) geeignet sind in einer Ausführungsform Verbindungen, welche neben einer oder mehreren primären Aminogruppen mindestens eine einen aktiven Wasserstoff tragende Reaktivgruppe in der Form einer Hydroxyl-, Mercapto- oder sekundäre Aminogruppe aufweisen. Beispiele für derartige Amine **B** mit mehr als einer aktiven Wasserstoff tragenden Reaktivgruppe sind

- mehr als eine sekundäre Aminogruppe und eine oder mehrere primäre Aminogruppen tragende aliphatische Amine, wie N,N'-Bis-(3-aminopropyl)-ethylendiamin, Triethylentetramin (TETA), Tetraethylenpentamin (TEPA), Pentaethylenhexamin und höhere Homologe linearer Polyethylenamine, N,N'-Bis-(3-aminopropyl)-ethylendiamin, Produkte aus der mehrfachen Cyanoethylierung oder Cyanobutylierung und anschliessender Hydrierung von primären Di- und Polyaminen mit mehreren primären Aminogruppen, wie N,N'-Bis-(3-aminopropyl)-ethylendiamin, N,N'-Bis-(3-aminopropyl)-1,4-diaminobutan, N,N'-Bis-(3-aminopropyl)-2-methyl-1,5-pentandiamin, N,N'-Bis-(3-amino-1-ethylpropyl)-2-methyl-1,5-pentandiamin sowie Polyethylenimine unterschiedlichen Polymerisationsgrades (Molmassen-Bereich 500 bis 1'000'000 g/mol), wie sie beispielsweise unter dem Handelsnamen Lupasol® von BASF in reiner Form oder als wässrige Lösungen erhältlich sind, wobei diese Polyethylenimine neben primären und sekundären auch tertiäre Aminogruppen enthalten;
- mehr als eine Hydroxylgruppe und eine oder mehrere primäre Aminogruppen tragende Hydroxyamine, insbesondere Derivate von polyalkoxylierten drei- oder höherwertigen Alkoholen oder von polyalkoxylierten Polyaminen, sowie Aminozucker, beispielsweise Glucosamin oder Galactosamin;
- mindestens eine Hydroxyl- und mindestens eine sekundäre Aminogruppen tragende Hydroxypolyamine aus der Cyanoethylierung oder Cyanobutylierung und anschliessender Hydrierung von Hydroxyaminen wie N-Hydroxyethyl-1,2-ethandiamin, N-Hydroxypropyl-1,2-ethandiamin, N-Hydroxyethyl-1,3-propandiamin, N3-Hydroxyethyl-1,3-pentandiamin.

Als Amin **B** der Formel (III) zur Umsetzung mit einem Aldehyd **ALD** der Formel (IV) besonders geeignet sind zum einen Amine **B1** der Formel (III a),

$$HX^{1a}\text{-}A^1\text{-}NH_2 \qquad \text{(III a)}$$

wobei

$X^{1a}$ für O oder S oder N-$R^{8a}$ oder N-$R^9$ steht,
wobei $R^{8a}$ entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
oder für einen Substituenten der Formel (III a') steht,

$-----B^1-NH_2$ (III a')

und $A^1$, $B^1$ und $R^9$ die bereits genannten Bedeutungen aufweisen.

Aus der Umsetzung von mindestens einem Amin **B1** der Formel (III a) mit mindestens einem Aldehyd **ALD** der Formel (IV) wird ein Aldimin der Formel (I a) erhalten.

Beispiele für Amine **B1** sind

- Verbindungen mit einer oder zwei primären aliphatischen und einer sekundären Aminogruppe, wie beispielsweise N-Methyl-1,2-ethandiamin, N-Ethyl-1,2-ethandiamin, N-Butyl-1,2-ethandiamin, N-Hexyl-1,2-ethandiamin, N-(2-Ethylhexyl)-1,2-ethandiamin, N-Cyclohexyl-1,2-ethandiamin, 4-Aminomethyl-piperidin, 3-(4-Aminobutyl)-piperidin, N-(2-Aminoethyl)piperazin, Diethylentriamin (DETA), Bis-hexamethylentriamin (BHMT), 3-(2-Aminoethyl)aminopropylamin; Di- und Triamine aus der Cyanoethylierung oder Cyanobutylierung und anschliessender Hydrierung von primären Mono- und Diaminen, beispielsweise N-Methyl-1,3-propandiamin, N-Ethyl-1,3-propandiamin, N-Butyl-1,3-propandiamin, N-Hexyl-1,3-propandiamin, N-(2-Ethylhexyl)-1,3-propandiamin, N-Dodecyl-1,3-propandiamin, N-Cyclohexyl-1,3-propandiamin, 3-Methylamino-1-pentylamin, 3-Ethylamino-1-pentylamin, 3-Butylamino-1-pentylamin, 3-Hexylamino-1-pentylamin, 3-(2-Ethylhexyl)-amino-1-pentylamin, 3-Dodecylamino-1-pentylamin, 3-Cyclohexylamino-1-pentylamin, Dipropylentriamin (DPTA), N3-(3-Aminopentyl)-1,3-pentandiamin, N5-(3-Aminopropyl)-2-methyl-1,5-pentandiamin, N5-(3-Amino-1-ethylpropyl)-2-methyl-1,5-pentandiamin, und Fettdiamine wie N-Cocoalkyl-1,3-propandiamin, N-Oleyl-1,3-propandiamin, N-Soyaalkyl-1,3-propandiamin, N-Talgalkyl-1,3-propandiamin oder N-($C_{16-22}$-Alkyl)-1,3-propandiamin, wie sie beispielsweise unter dem Handelsnamen Duomeen® von Akzo Nobel erhältlich sind; die Produkte aus der Michael-artigen Addition von aliphatischen primären Di- oder Triaminen mit Acrylnitril, Malein- oder Fumarsäurediestern, Citraconsäurediestern, Acryl- und Methacrylsäureestern, Acryl- und Methacrylsäureamiden und Itaconsäurediestern, umgesetzt im Molverhältnis 1:1;
- Hydroxyamine, wie beispielsweise 2-Aminoethanol, 2-Amino-1-propanol, 1-Amino-2-propanol, 3-Amino-1-propanol, 4-Amino-1-butanol, 4-Amino-2-butanol, 2-Amino-2-methylpropanol, 5-Amino-1-pentanol, 6-Amino-1-hexanol, 7-Amino-1-heptanol, 8-Amino-1-octanol, 10-Amino-1-decanol, 12-Amino-1-dodecanol, 4-(2-Aminoethyl)-2-hydroxyethylbenzol, 3-Aminomethyl-3,5,5-trimethyl-cyclohexanol; eine primäre Aminogruppe tragende Derivate von Glykolen wie Diethylenglykol, Dipropylenglykol, Dibutylenglykol und höheren Oligomeren und Polymeren dieser Glykole, beispielsweise 2-(2-Aminoethoxy)-ethanol, 2-(2-(2-Aminoethoxy)ethoxy)ethanol, α-(2-Hydroxymethylethyl)-ω-(2-aminomethylethoxy)-poly(oxy(methyl-1,2-ethandiyl)); eine Hydroxylgruppe und eine primäre Aminogruppen tragende Derivate von polyalkoxylierten drei- oder höherwertigen Alkoholen; Produkte aus der einfachen Cyanoethylierung und anschliessender Hydrierung von Glykolen, beispielsweise 3-(2-Hydroxyethoxy)-propylamin, 3-(2-(2-Hydroxyethoxy)-ethoxy)-propylamin und 3-(6-Hydroxyhexyloxy)-propylamin;
- Mercaptoamine, wie beispielsweise 2-Aminoethanthiol (Cysteamin), 3-Aminopropanthiol, 4-Amino-1-butanthiol, 6-Amino-1-hexanthiol, 8-Amino-1-octanthiol, 10-Amino-1-decanthiol und 12-Amino-1-dodecanthiol.

Als Amin **B1** bevorzugt sind N-Methyl-1,2-ethandiamin, N-Ethyl-1,2-ethandiamin, N-Cyclohexyl-1,2-ethandiamin, N-Methyl-1,3-propandiamin, N-Ethyl-1,3-propandiamin, N-Butyl-1,3-propandiamin, N-Cyclohexyl-1,3-propandiamin, 4-Aminomethyl-piperidin, 3-(4-Aminobutyl)-piperidin, DETA, DPTA, BHMT und Fettdiamine wie N-Cocoalkyl-1,3-propandiamin, N-Oleyl-1,3-propandiamin, N-Soyaalkyl-1,3-propandiamin und N-Talgalkyl-1,3-propandiamin; Produkte aus der Michael-artigen Additionsreaktion von aliphatischen primären Diaminen mit Malein- und Fumarsäurediestern, Acryl- und Methacrylsäureestern, Acryl- und Methacrylsäureamiden, bevorzugt mit Maleinsäurediestern, insbesondere Maleinsäuredimethyl-, -diethyl-, -dipropyl- und -dibutylester, und mit Acrylsäureestern, insbesondere Acrylsäuremethylester, umgesetzt im Molverhältnis 1:1; sowie aliphatische Hydroxy- oder Mercaptoamine, in denen die primäre Aminogruppe von der Hydroxyl- oder Mercaptogruppe durch eine Kette von mindestens 5 Atomen, oder durch einen Ring, getrennt sind, insbesondere 5-Amino-1-pentanol, 6-Amino-1-hexanol und höhere Homologe davon, 4-(2-Aminoethyl)-2-hydroxyethylbenzol, 3-Aminomethyl-3,5,5-trimethyl-cyclohexanol, 2-(2-Aminoethoxy)-ethanol, Triethylenglykol-monoamin und höhere Oligo- und Polymere davon, 3-(2-Hydroxyethoxy)-propylamin, 3-(2-(2-Hydroxyethoxy)-ethoxy)-propylamin und 3-(6-Hydroxyhexyloxy)-propylam in.

Als Amin **B1** besonders bevorzugt sind Amine, welche ausgewählt sind aus der Gruppe bestehend aus N-Methyl-1,2-ethandiamin, N-Ethyl-1,2-ethandiamin, N-Cyclohexyl-1,2-ethandiamin, N-Methyl-1,3-propandiamin, N-Ethyl-1,3-propandiamin, N-Butyl-1,3-propandiamin, N-Cyclohexyl-1,3-propandiamin, 4-Aminomethyl-piperidin, 3-(4-Aminobutyl)-piperidin, DETA, DPTA, BHMT, Fettdiamine, insbesondere N-Cocoalkyl-1,3-propandiamin, N-Oleyl-1,3-propandiamin, N-Soyaalkyl-1,3-propandiamin und N-Talgalkyl-1,3-propandiamin; 5-Amino-1-pentanol, 6-Amino-1-hexanol, 4-(2-Aminoethyl)-2-hydroxyethylbenzol, 3-Aminomethyl-3,5,5-trimethyl-cyclohexanol, 2-(2-Aminoethoxy)-ethanol, Triethylenglykol-monoamin, 3-(2-Hydroxyethoxy)-propylamin, 3-(2-(2-Hydroxyethoxy)-ethoxy)-propylamin und 3-(6-Hydroxyhexyloxy)-propylamin.

Als Amin **B1** am meisten bevorzugt sind Amine, welche ausgewählt sind aus der Gruppe bestehend aus 5-Amino-1-pentanol, 6-Amino-1-hexanol, 4-(2-Aminoethyl)-2-hydroxyethylbenzol, 3-Aminomethyl-3,5,5-trimethyl-cyclohexanol, 2-(2-Aminoethoxy)-ethanol, Triethylenglykol-monoamin, 3-(2-Hydroxyethoxy)-propylamin, 3-(2-(2-Hydroxyethoxy)-ethoxy)-propylamin und 3-(6-Hydroxyhexyloxy)-propylamin.

Als Amin **B** der Formel (III) zur Umsetzung mit einem Aldehyd **ALD** der Formel (IV) besonders geeignet sind zum anderen Amine **B2** der Formel (III b),

$$A^2[-NH_2]_t \qquad \text{(III b)}$$

wobei $A^2$ und t die bereits genannten Bedeutungen aufweisen.

Aus der Umsetzung von mindestens einem Amin **B2** der Formel (III b) mit mindestens einem Aldehyd **ALD** der Formel (IV) wird ein Aldimin der Formel (I b) erhalten.

Beispiele für Amine **B2** sind

- aliphatische, cycloaliphatische oder arylaliphatische Diamine, beispielsweise Ethylendiamin, 1,2-Propandiamin, 1,3-Propandiamin, 2-Methyl-1,2-propandiamin, 2,2-Dimethyl-1,3-propandiamin, 1,3-Butandiamin, 1,4-Butandiamin, 1,3-Pentandiamin (DAMP), 1,5-Pentandiamin, 1,5-Diamino-2-methylpentan (MPMD), 2-Butyl-2-ethyl-1,5-pentandiamin (C11-Neodiamin), 1,6-Hexandiamin, 2,5-Dimethyl-1,6-hexandiamin, 2,2,4- und 2,4,4-Trimethylhexamethylendiamin (TMD), 1,7-Heptandiamin, 1,8-Octandiamin, 1,9-Nonandiamin, 1,10-Decandiamin, 1,11-Undecandiamin, 1,12-Dodecandiamin, 1,2-, 1,3-und 1,4-Diaminocyclohexan, Bis-(4-aminocyclohexyl)-methan ($H_{12}$-MDA), Bis-(4-amino-3-methylcyclohexyl)-methan, Bis-(4-amino-3-ethylcyclohexyl)-methan, Bis-(4-amino-3,5-dimethylcyclohexyl)-methan, Bis-(4-amino-3-ethyl-5-methylcyclohexyl)-methan (M-MECA), 1-Amino-3-aminomethyl-3,5,5-trimethylcyclohexan (= Isophorondiamin oder IPDA), 2- und 4-Methyl-1,3-diaminocyclohexan und Mischungen davon, 1,3- und 1,4-Bis-(aminomethyl)-cyclohexan, 2,5(2,6)-Bis-(aminomethyl)-bicyclo[2.2.1]heptan (NBDA), 3(4),8(9)-Bis-(aminomethyl)-tricyclo[5.2.1.0$^{2,6}$]decan, 1,4-Diamino-2,2,6-trimethylcyclohexan (TMCDA), 1,8-Menthandiamin, 3,9-Bis-(3-aminopropyl)-2,4,8,10-tetraoxaspiro[5.5]undecan sowie 1,3- und 1,4-Xylylendiamin;
- Ethergruppen-haltige aliphatische Diamine, beispielsweise Bis-(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin und höhere Oligomere dieser Diamine, Bis-(3-aminopropyl)poly-tetrahydrofurane und andere Polytetrahydrofuran-diamine mit Molekulargewichten im Bereich von beispielsweise 350 bis 5200, sowie Polyoxyalkylen-Diamine. Letztere stellen typischerweise Produkte aus der Aminierung von Polyoxyalkylen-Diolen dar und sind beispielsweise erhältlich unter dem Namen Jeffamine® (von Huntsman), unter dem Namen Polyetheramin (von BASF) oder unter dem Namen PC Amine® (von Nitroil). Insbesondere geeignete Polyoxyalkylen-Diamine sind Jeffamine® D-230, Jeffamine® D-400, Jeffamine® D-2000, Jeffamine® D-4000, Jeffamine® XTJ-511, Jeffamine® ED-600, Jeffamine® ED-900, Jeffamine® ED-2003, Jeffamine® XTJ-568, Jeffamine® XTJ-569, Jeffamine® XTJ-523, Jeffamine® XTJ-536, Jeffamine® XTJ-542, Jeffamine® XTJ-559, Jeffamine® EDR-104, Jeffamine® EDR-148, Jeffamine® EDR-176; Polyetheramin D 230, Polyetheramin D 400 und Polyetheramin D 2000, PC Amine® DA 250, PC Amine® DA 400, PC Amine® DA 650 und PC Amine® DA 2000;
- aliphatische, cycloaliphatische oder arylaliphatische Triamine wie 4-Aminomethyl-1,8-octandiamin, 1,3,5-Tris-(aminomethyl)-benzol, 1,3,5-Tris-(aminomethyl)-cyclohexan, Tris-(2-aminoethyl)-amin, Tris-(2-aminopropyl)-amin, Tris-(3-aminopropyl)-amin;
- Polyoxyalkylen-Triamine, welche typischerweise Produkte aus der Aminierung von Polyoxyalkylen-Triolen darstellen und beispielsweise erhältlich sind unter dem Handelsnamen Jeffamine® (von Huntsman), unter dem Namen Polyetheramin (von BASF) oder unter dem Namen PC Amine® (von Nitroil), wie zum Beispiel Jeffamine® T-403, Jeffamine® T-3000, Jeffamine® T-5000; Polyetheramin T403, Polyetheramin T5000; und PC Amine® TA 403, PC Amine® TA 5000.
- aromatische Di- und Triamine, wie beispielsweise 1,2-, 1,3- und 1,4-Phenylendiamin, 2,4- und 2,6-Toluylendiamin (TDA), 3,4-Toluylendiamin, 3,5-Dimethylthio-2,4- und -2,6-toluylendiamin, 3,5-Diethyl-2,4- und -2,6-toluylendiamin (DETDA), 2,4,6-Triethyl-1,3-phenylendiamin, 2,4,6-Triisopropyl-1,3-phenylendiamin, 3-Ethyl-5-methyl-2,4-toluylendiamin, 3,5-Diisopropyl-2,4-toluylendiamin, 3,5-Bis-(1-methylpropyl)-2,4-toluylendiamin, 3,5-Bis-(tert.butyl)-2,4-toluylendiamin, 3-Ethyl-5-isopropyl-2,4-toluylendiamin, 5-Isopropyl-2,4-toluylendiamin, 5-(tert.Butyl)-2,4-toluylendiamin, 4,6-Bis-(1-methylpropyl)-1,3-phenylendiamin, 4-Isopropyl-6-(tert.butyl)-1,3-phenylendiamin, 4-Ethyl-6-isopropyl-1,3-phenylendiamin, 4-Ethyl-6-(2-methylpropyl)-1,3-phenylendiamin, 4-Ethyl-6-(1-methylpropyl)-1,3-phenylendiamin, 4-Ethyl-6-(2-methylpropyl)-1,3-phenylendiamin, 4-Isopropyl-6-(1-methylpropyl)-1,3-phenylendiamin, 4-(tert.Butyl)-6-(2-methylpropyl)-1,3-phenylendiamin, 4-Cyclopentyl-6-ethyl-1,3-phenylendiamin, 4-Cyclopentyl-6-isopropyl-1,3-phenylendiamin, 4,6-Dicyclopentyl-1,3-phenylendiamin, 3-Isopropyl-2,6-toluylendiamin, 2-Methylpropyl-(4-chloro-3,5-diaminobenzoat), tert.Butyl-(4-chloro-3,5-diaminobenzoat), 2,6-Diaminopyridin, Melamin, 4,4'-, 2,4'- und 2,2'-Diaminodiphenylmethan (MDA), 3,3'-Dimethyl-4,4'-diaminodiphenylmethan, 3,3'-Dichloro-4,4'-diaminodiphenylmethan (MOCA), 3,3',5,5'-Tetraethyl-4,4'-diaminodiphenylmethan (M-DEA), 3,3',5,5'-Tetraethyl-2,2'-dichloro-4,4'-diaminodiphenylmethan (M-CDEA), 3,3'-Diisopropyl-5,5'-dimethyl-4,4'-diaminodiphenylmethan (M-MIPA), 3,3',5,5'-Tetraisopropyl-4,4'-diaminodiphenylmethan (M-DIPA), 3,3',5,5'-Tetra-(1-methylpropyl)-4,4'-diaminodiphenylmethan, 3,3'-Dimethyl-5,5'-di-tert.butyl-4,4'-diaminodiphenylmethan, 3,3'-Di-tert.butyl-4,4'-diaminodiphenylmethan, 4,4'-Diaminodiphenylsulfon (DDS), 4-Amino-N-(4-aminophenyl)-benzolsulfonamid, 5,5'-Methylendianthranilsäure, Dimethyl-(5,5'-Methylendianthranilat), 1,3-Propylen-bis-(4-aminobenzoat), 1,4-Butylen-bis-(4-aminobenzoat), Polytetramethylenoxid-bis-(4-aminobenzoat) (erhältlich als Versalink® von Air Products) und 1,2-Bis-(2-aminophenylthio)-ethan.

- Polyamine mit primären aromatischen und primären aliphatischen Aminogruppen, wie insbesondere 4-Aminoethylanilin, 4-Aminomethylanilin, 4-[(4-Aminocyclohexyl)methyl]anilin, 2-Aminoethylanilin, 2-Aminomethylanilin, 2-[(4-Aminocyclohexyl)methyl]anilin und 4-[(2-Aminocyclohexyl)methyl]anilin.

Bevorzugt ist das Amin **B2** ausgewählt aus der Gruppe bestehend aus 1,6-Hexamethylendiamin, MPMD, DAMP, IPDA, TMD, 1,3-Xylylendiamin, 1,3-Bis-(aminomethyl)cyclohexan, Bis-(4-aminocyclohexyl)-methan, Bis-(4-amino-3-methylcyclohexyl)-methan, 3(4),8(9)-Bis-(aminomethyl)-tricyclo[5.2.1.0$^{2,6}$]-decan, 1,2-, 1,3- und 1,4-Diaminocyclohexan, 1,4-Diamino-2,2,6-trimethylcyclohexan, 3,6-Dioxaoctan-1,8-diamin, 4,7-Dioxadecan-1,10-diamin, 4-Aminomethyl-1,8-octandiamin, Polyoxyalkylen-Polyaminen mit zwei oder drei Aminogruppen, insbesondere die unter dem Handelsnamen Jeffamine® erhältlichen Typen D-230, D-400, D-2000, T-403 und T-5000 von Huntsman und dazu analoge Verbindungen von BASF oder Nitroil; 1,3- und 1,4-Phenylendiamin, 2,4- und 2,6-Toluylendiamin, 4,4'-, 2,4'- und 2,2'-Diaminodiphenylmethan, 3,3'-Dichlor-4,4'-diaminodiphenylmethan und Mischungen der genannten Polyamine.

Zur Herstellung eines Aldimins der Formel (I) wird weiterhin mindestens ein Aldehyd **ALD** der Formel (IV) eingesetzt,

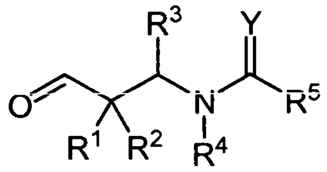
(IV)

wobei Y, $R^1$, $R^2$, $R^3$, $R^4$ und $R^5$ die bereits genannten Bedeutungen aufweisen.

In einer Ausführungsform ist ein Aldehyd **ALD** der Formel (IV) erhältlich als Produkt einer der Mannich-Reaktion analogen α-Aminoalkylierung, wie sie aus der Fachliteratur bekannt ist. Ein Aldehyd **Y1** der Formel (V), ein Aldehyd **Y2** der Formel (VI) und eine Verbindung **C** der Formel (VII) werden dabei unter Wasserabspaltung zu einem Aldehyd **ALD** der Formel (IV) umgesetzt.

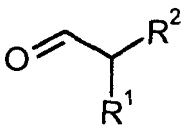
(V)

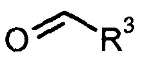
(VI)

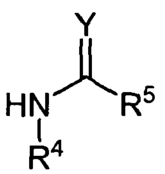
(VII)

In den Formeln (V), (VI) und (VII) weisen Y, $R^1$, $R^2$, $R^3$ und $R^4$ die bereits genannten Bedeutungen auf.

Diese Umsetzung kann entweder mit den freien Reagentien **Y1, Y2** und ***C*** gemäss den Formeln (V), (VI) und (VII) geführt werden, oder die Reagentien können teilweise oder vollständig in derivatisierter Form eingesetzt werden. In einer bevorzugten Ausführungsform wird die Umsetzung mit allen Reagentien in freier Form als Eintopfreaktion geführt

und der Aldehyd **ALD** nach erfolgter Umsetzung durch Destillation gereinigt. Bevorzugt werden dabei keine organischen Lösemittel eingesetzt.

Als Aldehyd **Y1** der Formel (V) geeignet sind insbesondere die folgenden Aldehyde: Isobutyraldehyd, 2-Methylbutyraldehyd, 2-Ethylbutyraldehyd, 2-Methylvaleraldehyd, 2-Ethylcapronaldehyd, Cyclopentancarboxaldehyd, Cyclohexancarboxaldehyd, 1,2,3,6-Tetrahydrobenzaldehyd, 2-Methyl-3-phenylpropionaldehyd, 2-Phenylpropionaldehyd und Diphenylacetaldehyd. Bevorzugt ist Isobutyraldehyd.

Als Aldehyd **Y2** der Formel (VI) geeignet sind insbesondere die folgenden Aldehyde: Formaldehyd, Acetaldehyd, Propionaldehyd, Butyraldehyd, Isobutyraldehyd, Phenylacetaldehyd und Glyoxylsäureester, insbesondere Glyoxylsäureethylester. Bevorzugt ist Formaldehyd.

Als Verbindung **C** der Formel (VII) geeignet sind einerseits Amide, insbesondere N-Methylformamid, N-Ethylformamid, N-Butylformamid, N-Methylacetamid, N-Ethylacetamid, N-Isopropylacetamid, N-Butylacetamid, N-N-(2-Ethylhexyl)acetamid, Cyclohexylacetamid, N-Benzylacetamid, N-Methylpropionamid, N-Methyl-butyramid, N-Methyl-2-ethylcapronamid, N-Methylbenzamid; weiterhin Lactame und Derivate davon, insbesondere 2-Pyrrolidon, 5-Methyl-2-pyrrolidon, Piperidin-2-on, ε-Caprolactam, 2-Azabicyclo[2.2.1]hept-5-en-3-on; weiterhin am Stickstoffatom monosubstituierte Carbamate und Derivate davon, insbesondere O-Ethyl-N-methylcarbamat, O-Ethyl-N-ethylcarbamat, O-Ethyl-N-propylcarbamat, O-Methyl-N-ethylcarbamat, O-Methyl-N-propylcarbamat, O-Methyl-N-butylcarbamat, Acetylurethan, N-Butylurethan, Oxazolidin-2-on, Oxazolidin-2,5-dion; weiterhin Imide und Derivate davon, insbesondere Pyrrolidin-2,5-dion (= Bernsteinsäureimid), 3,4-Dimethyl-pyrrolidin-2,5-dion, 3,3,4,4-Tetramethyl-pyrrolidin-2,5-dion, 3-Ethyl-3-methyl-pyrrolidin-2,5-dion, Piperidin-2,6-dion, 4,4-Dimethyl-piperidin-2,6-dion, 1,5,5-Trimethyl-imidazolidin-2,4-dion, Phthalimid, Methylphthalimid, Hexahydrophthalimid, Methylhexahydrophthalimid, 5,5-Dimethyl-1,3-oxazolidin-2,4-dion, Acetimid; weiterhin zu obigen Verbindungen analoge Substanzen mit Schwefel- anstelle von Sauerstoffatomen, insbesondere N-Methylthioacetamid, N-Butylthioacetamid, N-(2-Ethylhexyl)thioacetamid, N-Benzylthioacetamid, N-Methylbutyrthioamid, N-Methyl-(2-ethylcapronthioamid), N-Methylbenzthioamid, 2-Thiopyrrolidon, O-Ethyl-N-methyl-thiocarbamat, S-Ethyl-N-methyl-thiocarbamat, O-Methyl-N-ethyl-thiocarbamat, Thiazolidin-2-on und Thiazolidin-2,5-dion.

Bevorzugt ist die Verbindung **C** ausgewählt aus der Gruppe bestehend aus N-Methylformamid, N-Methylacetamid, N-Butylacetamid, N-(2-Ethylhexyl)-acetamid, N-Benzylacetamid, N-Methylbutyramid, N-Methyl-(2-ethylcapronamid), N-Methylbenzamid, O-Ethyl-N-methylcarbamat, 2-Pyrrolidon, Piperidin-2-on, ε-Caprolactam, Oxazolidin-2-on, Thiazolidin-2-on, Pyrrolidin-2,5-dion und Phthalimid.

In einer weiteren Ausführungsform ist ein Aldehyd **ALD** der Formel (IV) erhältlich ausgehend von einem Zwischenprodukt **ZW1** der Formel (VIII).

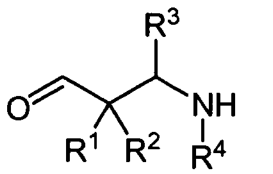

(VIII)

In der Formel (VIII) weisen $R^1$, $R^2$, $R^3$ und $R^4$ die bereits erwähnten Bedeutungen auf.

Das Zwischenprodukt **ZW1** der Formel (VIII) ist ebenfalls erhältlich als Produkt einer der Mannich-Reaktion analogen α-Aminoalkylierung, auf dieselbe Weise, wie zuvor für den Aldehyd **ALD** beschrieben, ausgehend von den bereits erwähnten Aldehyden **Y1** und **Y2,** wobei aber anstelle der Verbindung **C** der Formel (VII) ein primäres Amin der Formel $R^4$-$NH_2$ verwendet wird, wobei $R^4$ die bereits erwähnte Bedeutung aufweist, und wobei die Aldehyde **Y1** und **Y2** und das primäre Amin ungefähr im Molverhältnis 1:1:1 eingesetzt werden. Als primäres Amin zur Herstellung eines Zwischenproduktes **ZW1** geeignet sind insbesondere primäre aliphatische Amine, insbesondere Methylamin, Ethylamin, Propylamin, Isopropylamin, Butylamin, Isobutylamin, sek.-Butylamin, Hexylamin, Cyclohexylamin, Octylamin, 2-Ethyl-1-hexylamin, Benzylamin, 1- oder 2-Phenylethylamin und Decylamin.

Zur Herstellung eines Aldehyds **ALD** der Formel (IV) kann das Zwischenprodukt **ZW1** anschliessend mit einer Carbonsäure, bevorzugt in Form eines Carbonsäurechlorids, -esters oder -anhydrids, zum entsprechenden Amid umgesetzt werden, wobei als Carbonsäure gesättigte aliphatische oder cycloaliphatische Carbonsäuren geeignet sind, wie insbesondere Ameisensäure, Essigsäure, Propionsäure, Buttersäure, Isobuttersäure, Valeriansäure, Isovaleriansäure, Pivalinsäure, Capronsäure, Cyclohexancarbonsäure, Oenanthsäure, Caprylsäure, 2-Ethylhexansäure, Pelargonsäure, Caprinsäure, Neodecansäure, Undecansäure, Laurinsäure, Tridecansäure, Myristinsäure, Pentadecansäure, Palmitinsäure, Margarinsäure, Stearinsäure, Isostearinsäure und Arachinsäure; einfach oder mehrfach ungesättigte aliphatische Carbonsäuren wie insbesondere Palmitoleinsäure, Ölsäure, Erucasäure, Sorbinsäure, Linolsäure, Linolensäure, Elaeostearinsäure, Ricinolsäure und Ricinensäure; aromatische Carbonsäuren wie insbesondere Benzoesäure und die stellungsisomeren Tolylsäuren, Methoxybenzoesäuren und Nitrobenzoesäuren; sowie Chloride, Ester und Anhydride

der genannten Carbonsäuren; sowie weiterhin Anhydride von Dicarbonsäuren wie Phthalsäureanhydrid, 4-Methyl-phthalsäureanhydrid, Bernsteinsäureanhydrid, Maleinsäureanhydrid, Citraconsäureanhydrid, Hexahydrophthalsäureanhydrid, 4-Methylhexahydrophthalsäureanhydrid und 1,2,3,6- Tetrahydrophthalsäureanhydrid.

Zur Herstellung eines Aldehyds **ALD** der Formel (IV) kann das Zwischenprodukt **ZW1** weiterhin mit einem Carbonat oder bevorzugt einem Chlorameisensäureester, wie insbesondere Methyl-, Ethyl-, Propyl-, Isopropyl-, Butyl-, Isobutyl-, Neopentyl-, Hexyl-, Octyl-, 2-Ethylhexyl-, Benzyl-, Phenyl-, Tolyl- und Methoxyphenyl-chloroformiat, zum entsprechenden Urethan umgesetzt werden.

Zur Herstellung eines Aldehyds **ALD** der Formel (IV) kann das Zwischenprodukt **ZW1** weiterhin mit einem N,N-disubstituierten Carbamat oder einem N,N-disubstituierten Carbaminsäurechlorid, wie insbesondere N,N-Dimethyl-, N,N-Diethyl-, N,N-Diisopropyl-, N,N-Dibutyl-, N-Methyl-N-phenyl- und N,N-Diphenyl-carbamoylchlorid, sowie insbesondere 1-Pyrrolidin-, 1-Piperidin-, 1-Morpholin- und 4-Methyl-1-piperazin-carbonylchlorid, zum entsprechenden Harnstoff umgesetzt werden.

Zur Herstellung eines Aldehyds **ALD** der Formel (IV) kann das Zwischenprodukt **ZW1** weiterhin mit einer Thiocarbonsäure, wie insbesondere Thioessigsäure, Thiopropionsäure, Thiobenzoesäure, Thiotolylsäure oder Phenylthioessigsäure, bevorzugt in Form eines Säurechlorids, -esters oder -anhydrids, zum entsprechenden Thioamid umgesetzt werden.

Zur Herstellung eines Aldehyds **ALD** der Formel (IV) kann das Zwischenprodukt **ZW1** weiterhin mit einem (Di) thiocarbonat, einem Chlor(di)thioameisensäureester, einem N,N-disubstituierten Thiocarbamat oder einem N,N-disubstituierten Carbaminsäurechlorid, wie insbesondere O-Methyl-, O-Ethyl-, O-Phenyl- oder O-p-Tolyl-chloridothiocarbonat, S-Methyl-, S-Ethyl-, S-Propyl- oder S-Phenyl-chloridothiocarbonat, Phenylchloridodithiocarbonat, N,N-Dimethyl, N,N-Diethyl-, N-Methyl-N-phenyl- oder N,N-Diphenyl-thiocarbamoylchlorid, zum entsprechenden (Di)thiourethan oder -harnstoff umgesetzt werden.

Eine weitere Möglichkeit zur Herstellung eines Aldehyds **ALD** der Formel (IV) bietet die Umsetzung eines Aldehyds **Y1** der Formel (V), wie er bereits vorgängig genannt wurde, mit einer Verbindung der Formel (IX),

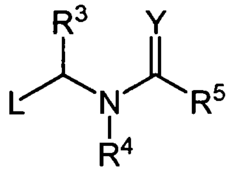

(IX)

wobei

L für einen Rest, ausgewählt aus der Gruppe bestehend aus einem Halogenatom, einer Alkoxygruppe, einer Carbonsäureestergruppe, einer über den Stickstoff gebundenen Urethangruppe, einer Dialkylaminogruppe und einer Di- oder Trialkylammoniumgruppe, steht,
und Y, $R^3$, $R^4$ und $R^5$ die bereits beschriebenen Bedeutungen aufweisen.

Als Verbindung der Formel (IX) geeignet sind insbesondere N-Chlor- oder N-Brommethyl-N-alkyl-carbamate wie N-Chlormethyl-N-methyl-carbaminsäureethylester, sowie N-Brommethyl-imide wie N-Brommethyl-phthalimid und N-Brommethyl-pyrrolidin-2,5-dion.

Die Aldehyde **ALD** der Formel (IV) haben eine Reihe von besonderen Eigenschaften. Sie weisen aufgrund ihrer chemischen Struktur auch bei relativ niedrigem Molekulargewicht keinen oder nur einen geringen, aminartigen Geruch auf. Sie sind also geruchsarm oder geruchsfrei. Weiterhin weisen sie in $\alpha$-Stellung zum Carbonyl-C-Atom kein Wasserstoffatom auf. Dadurch können ihre Aldehydgruppen nicht zu Enolgruppen tautomerisieren und sind damit gegenüber Isocyanatgruppen unreaktiv. Weiterhin sind sie, im Gegensatz zu $\beta$-Aminoaldehyden, nicht basisch, wodurch sie zusammen mit Isocyanatgruppen besonders gut lagerfähig sind, insbesondere auch zusammen mit aromatischen Isocyanatgruppen. Weiterhin weisen sie neben der Aldehydgruppe zusätzlich funktionelle Gruppen auf, welche zur Ausbildung von Wasserstoffbrücken-Bindungen fähig sind. Dies ist möglicherweise einer der Gründe dafür sein, dass sie in Wasserstoffbrücken ausbildenden Kunststoffzusammensetzungen, insbesondere in Polyurethanzusammensetzungen, sehr gut verträglich sind. Sie neigen dort nicht zu Migration oder Ausschwitzen und haben nur eine geringe weichmachende Wirkung, was oft sehr vorteilhaft ist.

Der Aldehyd **ALD** der Formel (IV) ist bevorzugt ausgewählt aus der Gruppe bestehend aus N-(2,2-Dimethyl-3-oxopropyl)-N-methylformamid, N-(2,2-Dimethyl-3-oxopropyl)-N-methylacetamid, N-(2,2-Dimethyl-3-oxopropyl)-N-butylacetamid, N-(2,2-Dimethyl-3-oxopropyl)-N-(2-ethylhexyl)acetamid, N-(2,2-Dimethyl-3-oxopropyl)-N-benzylacetamid, N-(2,2-Dimethyl-3-oxopropyl)-N-methylbutyramid, N-(2,2-Dimethyl-3-oxopropyl)-N-methyl-(2-ethylcapronamid),

N-(2,2-Dimethyl-3-oxopropyl)-N-methylbenzamid, O-Ethyl-N-(2,2-dimethyl-3-oxopropyl)-N-methylcarbamat, N-(2,2-Dimethyl-3-oxopropyl)-pyrrolidin-2-on, N-(2,2-Dimethyl-3-oxopropyl)-piperidin-2-on, N-(2,2-Dimethyl-3-oxopropyl)-azepan-2-on, N-(2,2-Dimethyl-3-oxopropyl)-oxazolidin-2-on, N-(2,2-Dimethyl-3-oxopropyl)-thiazolidin-2-on, N-(2,2-Dimethyl-3-oxopropyl)-pyrrolidin-2,5-dion und N-(2,2-Dimethyl-3-oxopropyl)-phthalimid.

Bevorzugt sind Aldimine der Formel (I), welche erhalten werden aus der Umsetzung von entweder mindestens einem Amin **B1** der Formel (III a) oder mindestens einem Amin **B2** der Formel (III b) und mindestens einem der genannten bevorzugten Aldehyde **ALD** der Formel (IV).

Eine weitere Möglichkeit, ein Aldimin der Formel (I) zu erhalten, geht aus von einem Zwischenprodukt **ZW2** der Formel (X),

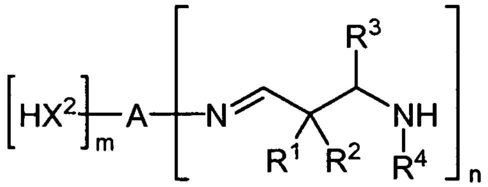

(X)

wobei

$X^2$ für O oder S steht,
und m, n, A, $R^1$, $R^2$, $R^3$ und $R^4$ die bereits genannten Bedeutungen aufweisen.

Zur Herstellung eines Aldimins der Formel (I) kann das Zwischenprodukt **ZW2** anstelle des Zwischenprodukts **ZW1** der Formel (VIII) entweder mit mindestens einer Carbon- bzw. Thiocarbonsäure, bevorzugt in Form eines Carbon- bzw. Thiocarbonsäurechlorids, -esters oder -anhydrids, zum entsprechenden Amid bzw. Thioamid, oder mit mindestens einem Carbonat bzw. Thiocarbonat, bevorzugt in Form eines Chlorameisensäure- bzw. Chlorthioameisensäureesters, zum entsprechenden Urethan bzw. Thiourethan umgesetzt werden, wobei dieselben Carbon- bzw. Thiocarbonsäuren und Carbonate bzw. Thiocarbonate geeignet sind, wie bereits bei der Umsetzung mit einem Zwischenprodukt **ZW1** beschrieben.

Das Zwischenprodukt **ZW2** wird insbesondere erhalten durch die Umsetzung eines entsprechenden Zwischenproduktes **ZW1** mit mindestens einem Amin **B** der Formel (III) in einem geeigneten Verhältnis, wobei aber Amine **B** mit sekundären Aminogruppen nicht geeignet sind. Bevorzugt für diese Umsetzung sind die genannten Amine **B2** und die genannten Amine **B1,** bei welchen $X^{1a}$ für O oder S, insbesondere für O, steht.

Diejenigen Ausführungsformen von Aldiminen der Formel (I), welche mindestens eine Gruppe HX aufweisen, können gegebenenfalls im Gleichgewicht stehen mit cyclischen Formen, wie sie in Formel (XI) beispielhaft für Aldimine der Formel (I) mit m = 1 gezeigt sind. Diese cyclischen Formen sind im Fall von Aminoaldiminen cyclische Aminale, beispielsweise Imidazolidine oder Tetrahydropyrimidine; im Fall von Hydroxyaldiminen cyclische Aminoacetale, beispielsweise Oxazolidine oder Tetrahydrooxazine; im Fall von Mercaptoaldiminen cyclische Thioaminale, beispielsweise Thiazolidine oder Tetrahydrothiazine.

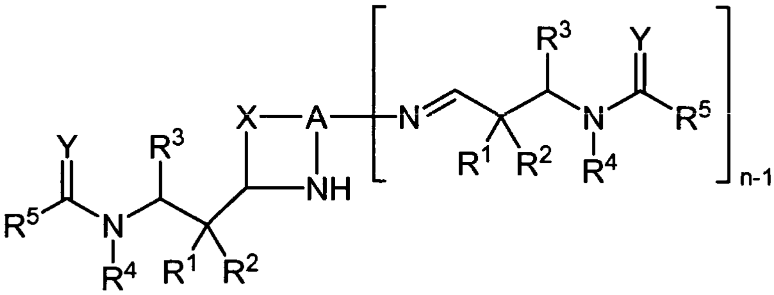

(XI)

In der Formel (XI) weisen n, A, $R^1$, $R^2$, $R^3$, $R^4$, $R^5$, X und Y die bereits genannten Bedeutungen auf.

Überraschenderweise neigen die meisten HX-Gruppen enthaltenden Aldimine der Formel (I) nicht zur Cyclisierung. Insbesondere für Aminoaldimine kann mittels IR- und NMR-spektroskopischen Methoden gezeigt werden, dass diese Verbindungen überwiegend in der offenkettigen, also der Aldimin-Form, vorliegen, während die cyclische, also die Aminal-Form, nicht oder nur in Spuren vorkommt. Auch Aldimine der Formel (I) abgeleitet von Hydroxy- und Mercaptoaminen, in denen die primäre Aminogruppe von der Hydroxylbeziehungsweise der Mercaptogruppe durch eine Kette von mindestens 5 Atomen oder durch einen Ring, getrennt sind, zeigen kaum Cyclisierung.

Bei den Aldiminen der Formel (I) handelt es sich um neue, bisher nicht beschriebenen Verbindungen mit vorteilhaften Eigenschaften. Sie enthalten tertiäre Aldiminogruppen, die also in $\alpha$-Stellung zur Carbonylgruppe stehenden C-Atom kein Wasserstoffatom aufweisen und deshalb nicht zu Enaminogruppen tautomerisieren können. Dadurch stellen diese Aldiminogruppen besonders gut geschützte ("blockierte") primäre Aminogruppen dar, welche unter Ausschluss von Feuchtigkeit mit gegenüber Aminogruppen reaktiven Verbindungen keine oder nur äusserst geringe Reaktivität zeigen. Weiterhin weisen die Aldimine der Formel (I) eine Amid-, Thioamid-, Urethan-, Thiourethan-, Harnstoff- oder Thioharnstoffgruppe auf. Die Aldimine der Formel (I) sind zusammen mit Isocyanatgruppen überraschend gut lagerfähig, insbesondere auch zusammen mit aromatischen Isocyanatgruppen. Möglicherweise rührt diese gute Lagerfähigkeit daher, dass die Stickstoffatome von Amid-, Thioamid-, Urethan-, Thiourethan-, Harnstoff- und Thioharnstoffgruppen kaum basisch sind. Weiterhin weisen die Aldimine der Formel (I) auch bei relativ niedrigem Molekulargewicht des zugrunde liegenden Aldehyds **ALD** keinen oder nur einen geringen, aminartigen Geruch auf. Weiterhin vorteilhaft ist die Tatsache, dass es sich bei den Aldiminen der Formel (I) meist um flüssige Verbindungen handelt.

Die Aldimine der Formel (I) sind unter geeigneten Bedingungen lagerstabil. Bei Zutritt von Feuchtigkeit können ihre Aldiminogruppen über Zwischenstufen formal zu Aminogruppen hydrolysieren, wobei die entsprechenden, zur Herstellung der Aldimine verwendeten Aldehyde **ALD** der Formel (IV) freigesetzt werden. Da diese Hydrolysereaktion reversibel ist und das chemische Gleichgewicht deutlich auf der Aldiminseite liegt, ist davon auszugehen, dass in Abwesenheit von gegenüber Aminen reaktiven Verbindungen nur ein Teil der Aldiminogruppen teilweise oder ganz hydrolysieren.

Die Aldimine der Formel (I) lassen sich sehr breit verwenden. Sie lassen sich zum Beispiel überall dort einsetzen, wo sie als Quelle von Aldehyden **ALD** der Formel (IV) oder von Aminen **B** der Formel (III) dienen können. Insbesondere können sie in der Funktion von geschützten Aminen, beziehungsweise geschützten Aldehyden, in Aldehyd- und/oder Amin-reaktiven Systemen eingesetzt werden und dort bei Bedarf gezielt entschützt werden. Insbesondere finden sie in Systemen Anwendung, in welchen Verbindungen vorhanden sind, welche mit primären Aminen reagieren. Die Entschützung erfolgt hydrolytisch, beispielsweise durch Kontakt mit Luftfeuchtigkeit oder Wasser, und lässt sich durch Säuren katalysieren.

Aldimine der Formel (I) mit m > 0 finden weiterhin Verwendung beim Aufbau von weiter funktionalisierten Reaktionsprodukten dieser Aldimine. So können Aldimine der Formel (I) mit m > 0 mit Verbindungen, welche mit der Gruppe HX Additionsreaktionen eingehen können, zu Additionsprodukten umgesetzt werden. Geeignete solche Additionsreaktionen eingehende Verbindungen tragen Reaktivgruppen wie zum Beispiel Isocyanatgruppen, Epoxidgruppen, Anhydridgruppen oder mehr oder weniger stark aktivierte Doppel- oder Dreifachbindungen wie (Meth)acrylatgruppen, Acrylamidgruppen, 1-Ethinylcarbonylgruppen, 1-Propinylcarbonylgruppen, Maleimidgruppen, Citraconimidgruppen, Vinylgruppen, Isopropenylgruppen oder Allylgruppen. Solche Aldiminogruppen tragenden Additionsprodukte lassen sich bei Bedarf zu Aldehyden **ALD** der Formel (IV) und Verbindungen mit primären Aminogruppen hydrolysieren und darauf für weitere Reaktionen nutzen, beispielsweise für Vernetzungsreaktionen.

Unter diesen Additionsprodukten besonders interessant sind Additionsprodukte **AV** der Formel (XII), welche erhältlich sind aus der Umsetzung von mindestens einem Polyisocyanat, insbesondere einem Polyisocyanat **P,** wie es weiter hinten in diesem Dokument ausführlich beschrieben ist, mit mindestens einem Aldimin der Formel (I a).

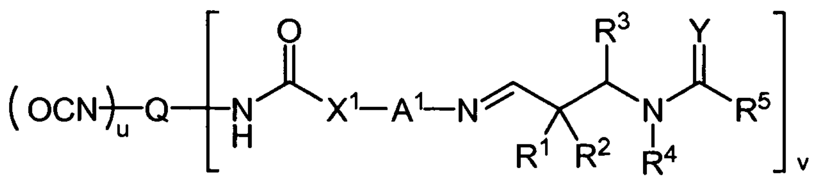

(XII)

Hierbei stehen

u für 0 oder 1 oder 2 oder 3,
v für 1 oder 2 oder 3 oder 4,
mit der Massgabe, dass (u+v) für 2 oder 3 oder 4 steht;
Q für den Rest eines (u+v) Isocyanatgruppen aufweisenden Polyisocyanates **P** nach Entfernung aller Isocyanatgruppen;
und $A^1$, $X^1$, Y, $R^1$, $R^2$, $R^3$, $R^4$ und $R^5$ weisen die bereits genannten Bedeutungen auf.

Aldimine der Formel (I) und Additionsprodukte **AV** der Formel (XII) eignen sich insbesondere zur Verwendung

in Zusammensetzungen basierend auf Isocyanaten. Sie lassen sich insbesondere als latente Härter oder als CoMonomere in ein- oder zweikomponentigen, Isocyanatgruppen aufweisenden Zusammensetzungen, welche als Klebstoff, Dichtstoff, Vergussmasse, Beschichtung, Bodenbelag, Anstrich, Lack, Primer oder Schaum angewendet werden, einsetzen.

Wie bereits erwähnt, enthalten die Aldimine der Formel (I) und ihre Additionsprodukte tertiäre, nicht zu Enaminogruppen tautomerisierende Aldiminogruppen, welche besonders gut geschützte primäre Aminogruppen darstellen. Zusammen mit Isocyanatgruppen aufweisenden Verbindungen können die Aldimine der Formel (I) unter Ausschluss von Feuchtigkeit lagerstabile, das heisst weitgehend viskositätskonstante, Mischungen bilden, wobei zu berücksichtigen ist, dass der in den Aldiminen (I) gegebenenfalls enthaltene aktive Wasserstoff mit den Isocyanatgruppen reagiert und dabei Additionsprodukte, wie beispielsweise die Additionsprodukte **AV** der Formel (XII), bildet. Insbesondere sind auch Mischungen, welche freie aromatische Isocyanatgruppen enthalten, lagerstabil.

Eine Zusammensetzung aus einer Isocyanatgruppen aufweisenden Verbindung und einem Aldimin der Formel (I) reagiert bei Kontakt mit Feuchtigkeit, insbesondere in Form von Luftfeuchtigkeit, unter Hydrolyse der Aldiminogruppen; die Isocyanatgruppen reagieren dabei mit den durch die Hydrolyse der Aldiminogruppen formal frei werdenden primären Aminogruppen zu Harnstoffgruppen, wobei ein Aldehyd **ALD** freigesetzt wird. Im Verhältnis zu den Aldiminogruppen überschüssige Isocyanatgruppen reagieren direkt mit Feuchtigkeit und bilden ebenfalls Harnstoffgruppen. Bei geeigneter Stöchiometrie zwischen Isocyanatgruppen und Aldiminogruppen härtet die Zusammensetzung als Ergebnis dieser Reaktionen aus; dieser Prozess wird auch als Vernetzung bezeichnet. Die Reaktion der Isocyanatgruppen mit den hydrolysierenden Aldiminogruppen muss dabei nicht notwendigerweise über freie Aminogruppen erfolgen. Selbstverständlich sind auch Reaktionen mit Zwischenstufen der Hydrolysereaktion möglich. Beispielsweise ist es denkbar, dass eine hydrolysierende Aldiminogruppe in der Form eines Halbaminals direkt mit einer Isocyanatgruppe reagiert.

Nach erfolgter Aushärtung verbleibt der freigesetzte Aldehyd **ALD** in der ausgehärteten Zusammensetzung. Er ist mit dieser ausgezeichnet verträglich, neigt kaum zu Migration oder Ausschwitzen und hat nur eine geringe weichmachende Wirkung, was oft sehr vorteilhaft ist.

Ein weiterer Gegenstand der Erfindung sind härtbare Zusammensetzungen, im Folgenden auch Polyurethanzusammensetzungen genannt, welche mindestens ein Polyisocyanat und mindestens ein Aldimin der Formel (I) enthalten. Falls das Aldimin der Formel (I) mindestens eine HX-Gruppe aufweist, kann es auch in Form eines Additionsprodukts, insbesondere als Additionsprodukt **AV** der Formel (XII), vorliegen.

Mit "Poly" beginnende Substanznamen wie Polyol, Polyisocyanat oder Polyaldehyd bezeichnen im vorliegenden Dokument Substanzen, die formal zwei oder mehr der in ihrem Namen vorkommenden funktionellen Gruppen pro Molekül enthalten.

Der Begriff "Polyisocyanat" umfasst im vorliegenden Dokument Verbindungen mit zwei oder mehr Isocyanatgruppen, unabhängig davon, ob es sich dabei um monomere Diisocyanate, oligomere Polyisocyanate oder Isocyanatgruppen aufweisende Polymere mit einem relativ hohen Molekulargewicht handelt.

Der Begriff "Polymer" umfasst im vorliegenden Dokument einerseits ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde. Der Begriff umfasst andererseits auch Derivate eines solchen Kollektivs von Makromolekülen aus Polyreaktionen, Verbindungen also, die durch Umsetzungen, wie beispielsweise Additionen oder Substitutionen, von funktionellen Gruppen an vorgegebenen Makromolekülen erhalten wurden und die chemisch einheitlich oder chemisch uneinheitlich sein können. Der Begriff umfasst im Weiteren auch sogenannte Prepolymere, das heisst reaktive oligomere Voraddukte, deren funktionelle Gruppen am Aufbau von Makromolekülen beteiligt sind.

Der Begriff "Polyurethanpolymer" umfasst sämtliche Polymere, welche nach dem sogenannten Diisocyanat-Polyadditions-Verfahren hergestellt werden. Dies schliesst auch solche Polymere ein, die nahezu oder gänzlich frei sind von Urethangruppen. Beispiele für Polyurethanpolymere sind Polyether-Polyurethane, Polyester-Polyurethane, Polyether-Polyharnstoffe, Polyharnstoffe, Polyester-Polyharnstoffe, Polyisocyanurate und Polycarbodiimide.

Die als Aldimin der Formel (I) geeigneten Aldimine und ihre bevorzugten Ausführungsformen wurden vorgängig im Detail beschrieben. Als Aldimin der Formel (I) bevorzugt sind die Aldimine der Formel (I a) und die Aldimine der Formel (I b). Besonders bevorzugt sind Aldimine der Formel (I a), bei denen $HX^1$ für eine Hydroxyl- oder Mercaptogruppe, insbesondere für eine Hydroxylgruppe, steht, sowie Aldimine der Formel (I b). Am meisten bevorzugt sind Aldimine der Formel (I b).

Die Polyurethanzusammensetzung kann einkomponentig oder zweikomponentig sein. Eine zweikomponentige Polyurethanzusammensetzung weist eine Komponente **K1,** welche mindestens ein Polyisocyanat **P,** wie es weiter hinten im Detail beschrieben ist, enthält, und eine Komponente **K2,** welche mindestens eine Isocyanat-reaktive Substanz enthält, auf. Aldimine der Formel (I) können bei einer zweikomponentigen Polyurethanzusammensetzung grundsätzlich in der Komponente **K2** vorhanden sein. Bei Aldiminen der Formel (I) mit einem Index von m=0, insbesondere bei Aldiminen der Formel (I b), kann dieses Aldimin Teil der Komponente **K1** und/oder **K2** sein, während Aldimine der Formel (I), wie beispielsweise das Aldimin der Formel (I a), mit einem Index von $m \neq 0$ nicht in der Komponente **K2** vorhanden

sein können, ohne dass eine Reaktion mit dem Polyisocyanat **P** erfolgt.

Als Polyurethanzusammensetzung bevorzugt ist eine einkomponentige feuchtigkeitshärtende Zusammensetzung, enthaltend

a) mindestens ein Polyisocyanat **P** und
b) mindestens ein Aldimin der Formel (I b), und/oder mindestens ein Additionsprodukt **AV** der Formel (XII).

Als "einkomponentig" wird im vorliegenden Dokument eine härtbare Zusammensetzung bezeichnet, bei welcher alle Bestandteile der Zusammensetzung vermischt im gleichen Gebinde gelagert werden, und welche bei Raumtemperatur über einen längeren Zeitraum lagerstabil ist, sich also in ihren Anwendungs- oder Gebrauchseigenschaften durch die Lagerung nicht oder nur unwesentlich verändert, und welche nach der Applikation durch die Einwirkung von Feuchtigkeit aushärtet. Als "zweikomponentig" wird entsprechend eine härtbare Zusammensetzung bezeichnet, bei welcher die zwei Komponenten, d.h. **K1** bzw. **K2,** jeweils in einem eigenen Gebinde lagerstabil gelagert werden können.

Das Polyisocyanat **P** ist in einer Ausführungsform ein Isocyanatgruppen aufweisendes Polyurethanpolymer **PUP**.

Der Begriff "Polyurethanpolymer" umfasst sämtliche Polymere, welche nach dem sogenannten Diisocyanat-Polyadditions-Verfahren hergestellt werden. Dies schliesst auch solche Polymere ein, die nahezu oder gänzlich frei sind von Urethangruppen. Beispiele für Polyurethanpolymere sind Polyether-Polyurethane, Polyester-Polyurethane, Polyether-Polyharnstoffe, Polyharnstoffe, Polyester-Polyharnstoffe, Polyisocyanurate und Polycarbodiimide.

Ein geeignetes Polyurethanpolymer **PUP** ist insbesondere erhältlich aus der Umsetzung von mindestens einem Polyol mit mindestens einem Polyisocyanat. Diese Umsetzung kann dadurch erfolgen, dass das Polyol und das Polyisocyanat mit üblichen Verfahren, beispielsweise bei Temperaturen von 50 °C bis 100 °C, gegebenenfalls unter Mitverwendung geeigneter Katalysatoren, zur Reaktion gebracht werden, wobei das Polyisocyanat so dosiert ist, dass dessen Isocyanatgruppen im Verhältnis zu den Hydroxylgruppen des Polyols im stöchiometrischen Überschuss vorhanden sind. Vorteilhaft ist das Polyisocyanat so dosiert, dass ein NCO/OH-Verhältnis von 1.3 bis 5, insbesondere eines von 1.5 bis 3, eingehalten wird. Unter dem "NCO/OH-Verhältnis" wird das Verhältnis der Anzahl der eingesetzten Isocyanatgruppen zu der Anzahl der eingesetzten Hydroxylgruppen verstanden. Bevorzugt verbleibt im Polyurethanpolymer **PUP** nach der Umsetzung aller Hydroxylgruppen des Polyols ein Gehalt an freien Isocyanatgruppen von 0.5 bis 15 Gewichts-%, besonders bevorzugt von 0.5 bis 5 Gewichts-%.

Gegebenenfalls kann das Polyurethanpolymer **PUP** unter Mitverwendung von Weichmachern hergestellt werden, wobei die verwendeten Weichmacher keine gegenüber Isocyanaten reaktive Gruppen enthalten.

Als Polyole für die Herstellung eines Polyurethanpolymers **PUP** können beispielsweise die folgenden handelsüblichen Polyole oder Mischungen davon eingesetzt werden:

- Polyoxyalkylenpolyole, auch Polyetherpolyole oder Oligoetherole genannt, welche Polymerisationsprodukte von Ethylenoxid, 1,2-Propylenoxid, 1,2- oder 2,3-Butylenoxid, Oxetan, Tetrahydrofuran oder Mischungen davon sind, eventuell polymerisiert mit Hilfe eines Startermoleküls mit zwei oder mehreren aktiven Wasserstoffatomen wie beispielsweise Wasser, Ammoniak oder Verbindungen mit mehreren OH- oder NH-Gruppen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, Bisphenol A, hydriertes Bisphenol A, 1,1,1-Trimethylolethan, 1,1,1-Trimethylolpropan, Glycerin, Anilin, sowie Mischungen der vorgenannten Verbindungen. Eingesetzt werden können sowohl Polyoxyalkylenpolyole, die einen niedrigen Ungesättigtheitsgrad aufweisen (gemessen nach ASTM D-2849-69 und angegeben in Milliequivalent Ungesättigtheit pro Gramm Polyol (mEq/g)), hergestellt beispielsweise mit Hilfe von sogenannten Double Metal Cyanide Complex-Katalysatoren (DMC-Katalysatoren), als auch Polyoxyalkylenpolyole mit einem höheren Ungesättigtheitsgrad, hergestellt beispielsweise mit Hilfe von anionischen Katalysatoren wie NaOH, KOH, CsOH oder Alkalialkoholaten.
  Besonders geeignet sind Polyoxyalkylendiole oder Polyoxyalkylentriole, insbesondere Polyoxyethylen- und Polyoxypropylendi- und -triole.
  Speziell geeignet sind Polyoxyalkylendiole und -triole mit einem Ungesättigtheitsgrad tiefer als 0.02 mEq/g und mit einem Molekulargewicht im Bereich von 1'000 - 30'000 g/mol, sowie Polyoxypropylendiole und -triole mit einem Molekulargewicht von 400 - 8'000 g/mol.
  Ebenfalls besonders geeignet sind sogenannte Ethylenoxid-terminierte ("EO-endcapped", ethylene oxide-endcapped) Polyoxypropylenpolyole. Letztere sind spezielle Polyoxypropylenpolyoxyethylenpolyole, die beispielsweise dadurch erhalten werden, dass reine Polyoxypropylenpolyole, insbesondere Polyoxypropylendiole und -triole, nach Abschluss der Polypropoxylierungsreaktion mit Ethylenoxid weiter alkoxyliert werden und dadurch primäre Hydroxylgruppen aufweisen.
- Styrol-Acrylnitril- oder Acrylnitril-Methylmethacrylat-gepfropfte Polyetherpolyole.

- Polyesterpolyole, auch Oligoesterole genannt, hergestellt nach bekannten Verfahren, insbesondere der Polykondensation von Hydroxycarbonsäuren oder der Polykondensation von aliphatischen und/oder aromatischen Polycarbonsäuren mit zwei- oder mehrwertigen Alkoholen.

Als Polyesterpolyole insbesondere geeignet sind solche, welche hergestellt sind aus zwei- bis dreiwertigen, insbesondere zweiwertigen, Alkoholen, wie beispielsweise Ethylenglykol, Diethylenglykol, Propylenglykol, Dipropylenglykol, Neopentylglykol, 1,4-Butandiol, 1,5-Pentandiol, 3-Methyl-1,5-hexandiol, 1,6-Hexandiol, 1,8-Octandiol, 1,10-Decandiol, 1,12-Dodecandiol, 1,12-Hydroxystearylalkohol, 1,4-Cyclohexandimethanol, Dimerfettsäurediol (Dimerdiol), Hydroxypivalinsäureneopentylglykolester, Glycerin, 1,1,1-Trimethylolpropan oder Mischungen der vorgenannten Alkohole, mit organischen Di- oder Tricarbonsäuren, insbesondere Dicarbonsäuren, oder deren Anhydride oder Ester, wie beispielsweise Bernsteinsäure, Glutarsäure, Adipinsäure, Trimethyladipinsäure, Korksäure, Azelainsäure, Sebacinsäure, Dodecandicarbonsäure, Maleinsäure, Fumarsäure, Dimerfettsäure, Phthalsäure, Phthalsäureanhydrid, Isophthalsäure, Terephthalsäure, Dimethylterephthalat, Hexahydrophthalsäure, Trimellithsäure und Trimellithsäureanhydrid, oder Mischungen der vorgenannten Säuren, sowie Polyesterpolyole aus Lactonen wie beispielsweise aus ε-Caprolacton und Startern wie den vorgenannten zwei- oder dreiwertigen Alkoholen.

Besonders geeignete Polyesterpolyole sind Polyesterdiole.

- Polycarbonatpolyole, wie sie durch Umsetzung beispielsweise der oben genannten - zum Aufbau der Polyesterpolyole eingesetzten - Alkohole mit Dialkylcarbonaten, Diarylcarbonaten oder Phosgen zugänglich sind.
- Mindestens zwei Hydroxylgruppen tragende Blockcopolymere, welche mindestens zwei verschiedene Blöcke mit Polyether-, Polyester- und/oder Polycarbonatstruktur der oben beschriebenen Art aufweisen, insbesondere Polyetherpolyesterpolyole.
- Polyacrylat- und Polymethacrylatpolyole.
- Polyhydroxyfunktionelle Fette und Öle, beispielsweise natürliche Fette und Öle, inbesondere Ricinusöl; oder durch chemische Modifizierung von natürlichen Fetten und Ölen gewonnene - sogenannte oleochemische - Polyole, beispielsweise die durch Epoxidierung ungesättigter Öle und anschliessender Ringöffnung mit Carbonsäuren bzw. Alkoholen erhaltenen Epoxypolyester bzw. Epoxypolyether, oder durch Hydroformylierung und Hydrierung ungesättigter Öle erhaltene Polyole; oder aus natürlichen Fetten und Ölen durch Abbauprozesse wie Alkoholyse oder Ozonolyse und anschliessender chemischer Verknüpfung, beispielsweise durch Umesterung oder Dimerisierung, der so gewonnenen Abbauprodukte oder Derivaten davon erhaltene Polyole. Geeignete Abbauprodukte von natürlichen Fetten und Ölen sind insbesondere Fettsäuren und Fettalkohole sowie Fettsäureester, insbesondere die Methylester (FAME), welche beispielsweise durch Hydroformylierung und Hydrierung zu Hydroxyfettsäureestern derivatisiert werden können.
- Polykohlenwasserstoffpolyole, auch Oligohydrocarbonole genannt, wie beispielsweise polyhydroxyfunktionelle Polyolefine, Polyisobutylene, Polyisoprene; polyhydroxyfunktionelle Ethylen-Propylen-, Ethylen-Butylen- oder Ethylen-Propylen-Dien-Copolymere, wie sie beispielsweise von der Firma Kraton Polymers hergestellt werden; polyhydroxyfunktionelle Polymere von Dienen, insbesondere von 1,3-Butadien, welche insbesondere auch aus anionischer Polymerisation hergestellt sein können; polyhydroxyfunktionelle Copolymere aus Dienen wie 1,3-Butadien oder Diengemischen und Vinylmonomeren wie Styrol, Acrylonitril, Vinylchlorid, Vinylacetat, Vinylalkohol, Isobutylen und Isopren, beispielsweise polyhydroxyfunktionelle Acrylonitril/Butadien-Copolymere, wie sie beispielsweise aus Epoxiden oder Aminoalkoholen und carboxylterminierten Acrylonitril/Butadien-Copolymeren (beispielsweise kommerziell erhältlich unter dem Namen Hypro® (früher Hycar®) CTBN und CTBNX und ETBN von Nanoresins AG, Deutschland, bzw. Emerald Performance Materials LLC) hergestellt werden können; sowie hydrierte polyhydroxyfunktionelle Polymere oder Copolymere von Dienen.

Diese genannten Polyole weisen bevorzugt ein mittleres Molekulargewicht von 250 - 30’000 g/mol, insbesondere von 400 - 20’000 g/mol, und weisen bevorzugt eine mittlere OH-Funktionalität im Bereich von 1.6 bis 3 auf.

Als Polyole bevorzugt sind Polyether-, Polyester-, Polycarbonat- und Polyacrylatpolyole, bevorzugt Di- und Triole. Besonders bevorzugt sind Polyetherpolyole, insbesondere Polyoxypropylen- und Polyoxypropylenpolyoxyethylenpolyole, sowie flüssige Polyesterpolyole und Polyetherpolyesterpolyole.

Zusätzlich zu diesen genannten Polyolen können kleine Mengen von niedrigmolekularen zwei- oder mehrwertigen Alkoholen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, hydriertes Bisphenol A, dimere Fettalkohole, 1,1,1-Trimethylolethan, 1,1,1-Trimethylolpropan, Glycerin, Pentaerythrit, Zuckeralkohole wie Xylit, Sorbit oder Mannit, Zucker wie Saccharose, andere höherwertige Alkohole, niedrigmolekulare Alkoxylierungsprodukte der vorgenannten zwei- und mehrwertigen Alkohole, sowie Mischungen der vorgenannten Alkohole bei der Herstellung des Polyurethanpolymers **PUP** mitverwendet werden. Ebenso können kleine Mengen an Polyolen mit einer

mittleren OH-Funktionalität von mehr als 3 mitverwendet werden, beispielsweise Zuckerpolyole.

Als Polyisocyanat für die Herstellung eines Isocyanatgruppen aufweisenden Polyurethanpolymers **PUP** werden aromatische oder aliphatische Polyisocyanate, insbesondere die Diisocyanate, eingesetzt.

Als aromatische Polyisocyanate eignen sich insbesondere monomere Di- oder Triisocyanate wie 2,4- und 2,6-Toluylendiisocyanat und beliebige Gemische dieser Isomeren (TDI), 4,4'-, 2,4'- und 2,2'-Diphenylmethandüsocyanat und beliebige Gemische dieser Isomeren (MDI), Gemische aus MDI und MDI-Homologen (polymeres MDI oder PMDI), 1,3- und 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diisocyanatodiphenyl (TODI), Dianisidindiisocyanat (DADI), 1,3,5-Tris-(isocyanatomethyl)-benzol, Tris-(4-isocyanatophenyl)-methan, Tris-(4-isocyanatophenyl)-thiophosphat, Oligomere und Polymere der vorgenannten Isocyanate, sowie beliebige Mischungen der vorgenannten Isocyanate. Bevorzugt sind MDI und TDI.

Als aliphatische Polyisocyanate eignen sich insbesondere monomere Di- oder Triisocyanate wie 1,4-Tetramethylendiisocyanat, 2-Methylpentamethylen-1,5-diisocyanat, 1,6-Hexamethylendiisocyanat (HDI), 2,2,4- und 2,4,4-Trimethyl-1,6-hexamethylendiisocyanat (TMDI), 1,10-Decamethylendiisocyanat, 1,12-Dodecamethylendiisocyanat, Lysin- und Lysinesterdiisocyanat, Cyclohexan-1,3- und -1,4-diisocyanat, 1-Methyl-2,4- und -2,6-diisocyanatocyclohexan und beliebige Gemische dieser Isomeren (HTDI oder $H_6$TDI), 1-Isocyanato-3,3,5-trimethyl-5-isocyanatomethyl-cyclohexan (=Isophorondiisocyanat oder IPDI), Perhydro-2,4'- und -4,4'-diphenylmethandiisocyanat (HMDI oder $H_{12}$MDI), 1,4-Diisocyanato-2,2,6-trimethylcyclohexan (TMCDI), 1,3- und 1,4-Bis-(isocyanatomethyl)-cyclohexan, m- und p-Xylylendiisocyanat (m- und p-XDI), m- und p-Tetramethyl-1,3- und -1,4-xylylendiisocyanat (m- und p-TMXDI), Bis-(1-Isocyanato-1-methylethyl)-naphthalin, Dimer- und Trimerfettsäureisocyanate wie 3,6-Bis-(9-isocyanatononyl)-4,5-di-(1-heptenyl)-cyclohexen (Dimeryldiisocyanat), $\alpha,\alpha,\alpha',\alpha',\alpha'',\alpha''$-Hexamethyl-1,3,5-mesitylentriisocyanat, Oligomere und Polymere der vorgenannten Isocyanate, sowie beliebige Mischungen der vorgenannten Isocyanate. Bevorzugt sind HDI und IPDI.

Bevorzugt sind Polyurethanpolymere **PUP** mit aromatischen Isocyanatgruppen.

Das Polyisocyanat **P** ist in einer weiteren Ausführungsform ein Polyisocyanat **PI** in der Form eines monomeren Di- oder Triisocyanates oder eines Oligomeren eines monomeren Diisocyanates oder eines Derivates eines monomeren Diisocyanates, wobei als monomeres Di- oder Triisocyanat insbesondere die vorgenannten aromatischen und aliphatischen Di- und Triisocyanate geeignet sind.

Als Polyisocyanat **PI** besonders geeignet sind Oligomere oder Derivate von monomeren Diisocyanaten, insbesondere von HDI, IPDI, TDI und MDI. Kommerziell erhältliche Typen sind insbesondere HDI-Biurete, beispielsweise als Desmodur® N 100 und N 3200 (von Bayer), Tolonate® HDB und HDB-LV (von Rhodia) und Duranate® 24A-100 (von Asahi Kasei); HDI-Isocyanurate, beispielsweise als Desmodur® N 3300, N 3600 und N 3790 BA (alle von Bayer), Tolonate® HDT, HDT-LV und HDT-LV2 (von Rhodia), Duranate® TPA-100 und THA-100 (von Asahi Kasei) und Coronate® HX (von Nippon Polyurethane); HDI-Uretdione, beispielsweise als Desmodur® N 3400 (von Bayer); HDI-Iminooxadiazindione, beispielsweise als Desmodur® XP 2410 (von Bayer); HDI-Allophanate, beispielsweise als Desmodur® VP LS 2102 (von Bayer); IPDI-Isocyanurate, beispielsweise in Lösung als Desmodur® Z 4470 (von Bayer) oder in fester Form als Vestanat® T1890/100 (von Degussa); TDI-Oligomere, beispielsweise als Desmodur® IL (von Bayer); sowie gemischte Isocyanurate auf Basis TDI / HDI, zum Beispiel als Desmodur® HL (von Bayer). Weiterhin besonders geeeignet sind bei Raumtemperatur flüssige Formen von MDI (sogenanntes "modifiziertes MDI"), welche Gemische von MDI mit MDI-Derivaten, wie beispielsweise MDI-Carbodiimiden bzw. MDI-Uretoniminen oder MDI-Urethanen darstellen, bekannt beispielsweise unter Handelsnamen wie Desmodur® CD, Desmodur® PF, Desmodur® PC (alle von Bayer), sowie Gemische aus MDI und MDI-Homologen (polymeres MDI oder PMDI), erhältlich unter Handelsnamen wie Desmodur® VL, Desmodur® VL50, Desmodur® VL R10, Desmodur® VL R20 und Desmodur® VKS 20F (alle von Bayer), Isonate® M 309, Voranate® M 229 und Voranate® M 580 (alle von Dow) oder Lupranat® M 10 R (von BASF).

Die vorgenannten oligomeren Polyisocyanate **PI** stellen in der Praxis üblicherweise Gemische von Substanzen mit unterschiedlichen Oligomerisationsgraden und/oder chemischen Strukturen dar. Vorzugsweise weisen sie eine mittlere NCO-Funktionalität von 2.1 bis 4.0 auf und enthalten insbesondere Isocyanurat-, Iminooxadiazindion-, Uretdion-, Urethan-, Biuret-, Allophanat-, Carbodiimid-, Uretonimin- oder Oxadiazintrion-Gruppen. Bevorzugt weisen diese Oligomere einen niedrigen Gehalt an monomeren Diisocyanaten auf.

Als Polyisocyanat **PI** bevorzugt sind bei Raumtemperatur flüssige Formen von MDI, sowie die Oligomeren von HDI, IPDI und TDI, insbesondere die Isocyanurate und die Biurete.

In einer weiteren Ausführungsform ist das Polyisocyanat **P** eine Mischung bestehend aus mindestens einem Polyurethanpolymer **PUP** und mindestens einem Polyisocyanat **PI,** wie sie vorgängig beschrieben wurden.

Bevorzugt liegt das Polyisocyanat **P** in Form eines aromatische Isocyanatgruppen aufweisenden Polyurethanpolymers **PUP** vor.

Üblicherweise ist das Polyisocyanat **P** in einer Menge von 5 bis 95 Gewichts-%, bevorzugt in einer Menge von 10 bis 90 Gewichts-%, bezogen auf die gesamte Zusammensetzung, vorhanden. In gefüllten Zusammensetzungen, d.h. Zusammensetzungen, welche einen Füllstoff enthalten, ist das Polyisocyanat **P** bevorzugt in einer Menge von 5 bis 60 Gewichts-%, insbesondere 10 bis 50 Gewichts-%, bezogen auf die gesamte Zusammensetzung, vorhanden.

Die einkomponentige feuchtigkeitshärtende Zusammensetzung umfasst neben mindestens einem Polyisocyanat **P** weiterhin mindestens ein Aldimin der Formel (I b) und/oder ein Additionsprodukt **AV** der Formel (XII), wobei letzteres gegebenenfalls in situ aus mindestens einem Aldimin der Formel (I a) und dem Polyisocyanat **P** gebildet wird.

In der einkomponentigen feuchtigkeitshärtenden Zusammensetzung beträgt das Verhältnis zwischen der Summe aus der Anzahl Aldiminogruppen und HX-Gruppen und der Anzahl Isocyanatgruppen vorteilhaft 0.1 bis 1.1, bevorzugt 0.2 bis 0.9, insbesondere 0.5 bis 0.9.

Gegebenenfalls enthält die einkomponentige feuchtigkeitshärtende Zusammensetzung weitere Bestandteile, insbesondere die in Polyurethanzusammensetzungen üblicherweise eingesetzten Hilfs- und Zusatzstoffe, beispielsweise die folgenden:

- Weichmacher, insbesondere Carbonsäureester wie Phthalate, insbesondere Dioctylphthalat, Diisononylphthalat oder Diisodecylphthalat, Adipate, insbesondere Dioctyladipat, Azelate und Sebacate, organische Phosphor- und Sulfonsäureester oder Polybutene;
- nicht-reaktive thermoplastische Polymere, wie beispielsweise Homo- oder Copolymere von ungesättigten Monomeren, insbesondere aus der Gruppe umfassend Ethylen, Propylen, Butylen, Isobutylen, Isopren, Vinylacetat und Alkyl(meth)acrylate, insbesondere Polyethylene (PE), Polypropylene (PP), Polyisobutylene, Ethylenvinylacetat-Copolymere (EVA) und ataktische Poly-$\alpha$-Olefine (APAO);
- Lösemittel;
- anorganische und organische Füllstoffe, insbesondere gemahlene oder gefällte Calciumcarbonate, welche gegebenenfalls mit Fettsäuren, insbesondere Stearaten, beschichtet sind, Baryt ($BaSO_4$, auch Schwerspat genannt), Quarzmehle, calcinierte Kaoline, Aluminiumoxide, Aluminiumhydroxide, Kieselsäuren, insbesondere hochdisperse Kieselsäuren aus Pyrolyseprozessen, Russe, insbesondere industriell hergestellte Russe (im Folgenden als "Russ" bezeichnet), PVC-Pulver oder Hohlkugeln;
- Fasern, beispielsweise aus Polyethylen;
- Pigmente, beispielsweise Titandioxid oder Eisenoxide;
- Katalysatoren, welche die Hydrolyse der Aldiminogruppen beschleunigen, insbesondere Säuren, insbesondere organische Carbonsäuren wie Benzoesäure, Salicylsäure oder 2-Nitrobenzoesäure, organische Carbonsäureanhydride wie Phthalsäureanhydrid, Hexahydrophthalsäureanhydrid und Hexahydromethylphthalsäureanhydrid, Silylester von organischen Carbonsäuren, organische Sulfonsäuren wie Methansulfonsäure, p-Toluolsulfonsäure oder 4-Dodecylbenzolsulfonsäure, Sulfonsäureester, andere organische oder anorganische Säuren, oder Mischungen der vorgenannten Säuren und Säureester;
- Katalysatoren, welche die Reaktion der Isocyanatgruppen beschleunigen, insbesondere Organozinnverbindungen wie Dibutylzinndiacetat, Dibutylzinndilaurat, Dibutylzinndichlorid, Dibutylzinndiacetylacetonat und Dioctylzinndilaurat, Bismutverbindungen wie Bismuttrioctoat und Bismuttris(neodecanoat), und tertiäre Aminogruppen enthaltende Verbindungen wie 2,2'-Dimorpholinodiethylether und 1,4-Diazabicyclo[2.2.2]octan;
- Rheologie-Modifizierer wie insbesondere Verdickungsmittel oder Thixotropiermittel, zum Beispiel Harnstoffverbindungen, Polyamidwachse, Bentonite oder pyrogene Kieselsäuren;
- blockierte Amine, beispielsweise in Form von Ketiminen, Oxazolidinen, Enaminen oder anderen Aldiminen;
- Trocknungsmittel, wie beispielsweise Molekularsiebe, Calciumoxid, hochreaktive Isocyanate wie p-Tosylisocyanat, Orthoameisensäureester, Alkoxysilane wie Tetraethoxysilan, Organoalkoxysilane wie Vinyltrimethoxysilan, und Organoalkoxysilane, welche in $\alpha$-Stellung zur Silangruppe eine funktionelle Gruppe aufweisen;
- Haftvermittler, insbesondere Organoalkoxysilane ("Silane") wie beispielsweise Epoxysilane, Vinylsilane, (Meth) acrylsilane, Isocyanatosilane, Carbamatosilane, Alkylsilane, S-(Alkylcarbonyl)-mercaptosilane und Aldiminosilane, sowie oligomere Formen dieser Silane;
- Stabilisatoren gegen Wärme, Licht- und UV-Strahlung;
- flammhemmende Substanzen;
- oberflächenaktive Substanzen wie insbesondere Netzmittel, Verlaufsmittel, Entlüftungsmittel oder Entschäumer;
- Biozide wie beispielsweise Algizide, Fungizide oder das Pilzwachstum hemmende Substanzen.

Es ist vorteilhaft, beim Einsatz solcher weiteren Bestandteile darauf zu achten, dass diese die Lagerstabilität der Zusammensetzung nicht stark beeinträchtigen. Das heisst, dass diese Bestandteile während der Lagerung die zur Vernetzung führenden Reaktionen, wie Hydrolyse der Aldiminogruppen oder Vernetzung der Isocyanatgruppen, nicht in signifikantem Ausmass auslösen dürfen. Insbesondere bedeutet dies, dass all diese Bestandteile kein oder höchstens Spuren von Wasser enthalten sollten. Es kann sinnvoll sein, gewisse Bestandteile vor dem Einmischen in die Zusammensetzung chemisch oder physikalisch zu trocknen.

Bevorzugt enthält die einkomponentige feuchtigkeitshärtende Zusammensetzung mindestens einen Katalysator. Der Katalysator ist insbesondere eine der genannten Säuren, wie Benzoesäure oder Salicylsäure, oder eine der genannten Metallverbindungen, oder eines der genannten tertiären Amine. Es kann durchaus vorteilhaft sein, unterschiedliche Katalysatoren, bzw. unterschiedliche Katalysatorenarten, einzusetzen.

Die beschriebene einkomponentige feuchtigkeitshärtende Zusammensetzung wird unter Ausschluss von Feuchtigkeit hergestellt und aufbewahrt. Sie ist lagerstabil, d.h. sie kann unter Ausschluss von Feuchtigkeit in einer geeigneten Verpackung oder Anordnung, wie beispielsweise einem Fass, einem Eimer, einem Beutel, einer Kartusche oder einer Flasche über einen Zeitraum von beispielsweise mehreren Monaten aufbewahrt werden, ohne dass sie sich in ihren Anwendungseigenschaften oder in ihren Eigenschaften nach der Aushärtung in einem für ihren Gebrauch relevanten Ausmass verändert. Üblicherweise wird die Lagerstabilität über die Messung der Viskosität oder der Auspresskraft ermittelt.

Wie bereits erwähnt, haben die in der Zusammensetzung vorhandenen Aldiminogruppen die Eigenschaft, bei Kontakt mit Feuchtigkeit formal zu einem Aldehyd **ALD** der Formel (IV) und einem Amin **B** der Formel (III) zu hydrolysieren, wobei letzteres mit den Isocyanatgruppen reagiert. Im Verhältnis zu den Aldiminogruppen überschüssige Isocyanatgruppen reagieren direkt mit Feuchtigkeit und bilden ebenfalls Harnstoffgruppen. Als Ergebnis dieser Reaktionen härtet die Zusammensetzung zu einem festen Material aus; dieser Prozess wird auch als Vernetzung bezeichnet.

Die für die Aushärtung benötigte Feuchtigkeit kann entweder aus der Luft stammen (Luftfeuchtigkeit), oder aber die Zusammensetzung kann mit einer Wasser enthaltenden Komponente in Kontakt gebracht werden, zum Beispiel durch Bestreichen, beispielsweise mit einem Abglättmittel, oder durch Besprühen, oder es kann der Zusammensetzung bei der Applikation eine Wasser enthaltende Komponente zugesetzt werden, zum Beispiel in Form einer wasserhaltigen Paste, die, beispielsweise über einen Statikmischer, eingemischt wird.

Bevorzugt wird die Zusammensetzung mittels Luftfeuchtigkeit ausgehärtet.

Die einkomponentige feuchtigkeitshärtende Zusammensetzung härtet im Allgemeinen ohne die Bildung von Blasen aus. Die Aushärtungsgeschwindigkeit lässt sich über die Art und Menge eines oder mehrerer gegebenenfalls vorhandener Katalysatoren, über die bei der Aushärtung herrschende Temperatur sowie über die Luftfeuchtigkeit bzw. die Menge zugesetzten Wassers beeinflussen.

Die beschriebene einkomponentige feuchtigkeitshärtende Zusammensetzung weist eine Reihe von Vorteilen auf. Sie verfügt einerseits über eine gute Lagerstabilität; in einem geeigneten Gebinde unter Ausschluss von Feuchtigkeit kann sie während mehreren Monaten bis zu einem Jahr aufbewahrt werden und bleibt während dieser Zeit in der vorgesehenen Weise verwendbar. Bei Kontakt mit Feuchtigkeit härtet die Zusammensetzung andererseits schnell und vollständig zu einem hochelastischen, weitgehend klebfreien Material mit hoher Festigkeit, Dehnung und hohen Elastizitätsmodulen aus. Der bei der Aushärtung freigesetzte Aldehyd **ALD** der Formel (IV) ist in der Zusammensetzung sehr gut verträglich. Er weist nur eine geringe weichmachende Wirkung auf die ausgehärtete Zusammensetzung auf und neigt weder zum Ausschwitzen noch zum Migrieren. Auch bei einem relativ tiefen Molekulargewicht des bei der Aushärtung freigesetzten Aldehyds **ALD** entsteht vor, während und nach der Aushärtung wenig oder gar kein Geruch, was für viele Anwendungen von solchen Zusammensetzungen, insbesondere in Innenräumen, eine Voraussetzung ist.

Die beschriebenen Zusammensetzungen können als Klebstoff, Dichtstoff, Beschichtung, Bodenbelag, Vergussmasse, Anstrich, Lack, Primer oder Schaum, insbesondere als Klebstoff, Dichtstoff, Beschichtung oder Bodenbelag, verwendet werden.

Speziell geeignet sind sie für Anwendungen, bei welchen kaum oder gar kein Geruch toleriert wird, und bei welchen elastische Eigenschaften bei relativ hohen Elastizitätsmodulen gefordert sind.

Ein weiterer Aspekt der vorliegenden Erfindung betrifft ein Verfahren zum Verkleben von einem Substrat **S1** mit einem Substrat **S2,** welches die Schritte umfasst:

i) Applikation der einkomponentigen feuchtigkeitshärtenden Zusammensetzung auf ein Substrat **S1;**

ii) Kontaktieren der applizierten Zusammensetzung mit einem Substrat **S2** innerhalb der Offenzeit der Zusammensetzung;

oder

i') Applikation der einkomponentigen feuchtigkeitshärtenden Zusammensetzung auf ein Substrat **S1** und auf ein Substrat **S2**;

ii') Kontaktieren der applizierten Zusammensetzung miteinander innerhalb der Offenzeit der Zusammensetzung;

wobei das Substrat **S2** aus dem gleichen oder einem unterschiedlichen Material wie das Substrat **S1** besteht.

Als "Offenzeit" wird in diesem Dokument die Zeit bezeichnet, während der die Zusammensetzung verarbeitet werden kann, nachdem die Isocyanatgruppen des Polyisocyanats mit Feuchtigkeit in Kontakt gekommen sind.

Ein weiterer Aspekt der vorliegenden Erfindung betrifft ein Verfahren zum Abdichten. Dieses umfasst den Schritt:

i'') Applikation der einkomponentigen feuchtigkeitshärtenden Zusammensetzung zwischen ein Substrat **S1** und ein Substrat **S2,** so dass die Zusammensetzung mit dem Substrat **S1** und dem Substrat **S2** in Kontakt steht;

wobei das Substrat **S2** aus dem gleichen oder einem unterschiedlichen Material wie das Substrat **S1** besteht.

Üblicherweise wird der Dichtstoff in eine sogenannte Fuge eingepresst.

Ein weiterer Aspekt der vorliegenden Erfindung betrifft ein Verfahren zum Beschichten eines Substrates **S1**. Dieses umfasst den Schritt:

i''') Applikation der einkomponentigen feuchtigkeitshärtenden Zusammensetzung auf ein Substrat **S1** innerhalb der Offenzeit der Zusammensetzung.

In diesen Verfahren sind geeignete Substrate **S1** und/oder **S2** insbesondere

- Glas, Glaskeramik, Beton, Mörtel, Backstein, Ziegel, Gips und Natursteine wie Granit oder Marmor;
- Metalle oder Legierungen wie Aluminium, Stahl, Eisen, Buntmetalle, verzinkte Metalle;
- Leder, Textilien, Papier, Holz, mit Harz gebundene Holzwerkstoffe, Harz-Textil-Verbundwerkstoffe und weitere sogenannte Polymer-Composites;
- Kunststoffe wie Polyvinylchlorid (Hart- und Weich-PVC), AcrylonitrilButadien-Styrol-Copolymere (ABS), SMC (Sheet Moulding Compounds), Polycarbonat (PC), Polyamid (PA), Polyester, Poly(methylmethacrylat) (PMMA), Polyester, Epoxidharze, Polyurethane (PUR), Polyoxymethylen (POM), Polyolefine (PO), Polyethylen (PE) oder Polypropylen (PP), Ethylen/Propylen-Copolymere (EPM) und Ethylen/Propylen/Dien-Terpolymere (EPDM), wobei die Kunststoffe bevorzugt mittels Plasma, Corona oder Flammen oberflächenbehandelt sein können;
- beschichtete Substrate wie pulverbeschichtete Metalle oder Legierungen; sowie Farben und Lacke.

Die Substrate können bei Bedarf vor dem Applizieren der Zusammensetzung vorbehandelt werden. Derartige Vorbehandlungen umfassen insbesondere physikalische und/oder chemische Reinigungsverfahren, beispielsweise Schleifen, Sandstrahlen, Bürsten oder dergleichen, oder Behandeln mit Reinigern oder Lösemitteln oder das Aufbringen eines Haftvermittlers, einer Haftvermittlerlösung oder eines Primers.

Die Applikation der Zusammensetzung kann in einem breiten Temperaturspektrum erfolgen. Beispielsweise kann die Zusammensetzung bei Raumtemperatur appliziert werden. Die Zusammensetzung kann aber auch bei tieferen wie auch bei höheren Temperaturen appliziert werden.

Aus diesen beschriebenen Verfahren zum Verkleben, Abdichten bzw. Beschichten - beziehungsweise aus der Verwendung einer der beschriebenen Zusammensetzungen als Klebstoff, Dichtstoff, Vergussmasse, Beschichtung, Bodenbelag, Anstrich, Lack, Primer oder Schaum - entsteht ein Artikel.

Dieser Artikel ist insbesondere ein Bauwerk, insbesondere ein Bauwerk des Hoch- oder Tiefbaus, oder ein industrielles Gut oder ein Konsumgut, insbesondere ein Fenster, eine Haushaltmaschine, oder ein Transportmittel, insbesondere ein Fahrzeug zu Wasser oder zu Lande, bevorzugt ein Automobil, ein Bus, ein Lastkraftwagen, ein Zug oder ein Schiff, oder ein Anbauteil eines Transportmittels, oder ein Artikel der Möbel-, Textil- oder Verpackungsindustrie.

**Beispiele**

Beschreibung der Messmethoden

**Infrarotspektren** wurden auf einem FT-IR Gerät 1600 von Perkin-Elmer gemessen (horizontale ATR-Messeinheit mit ZnSe-Kristall). Flüssige Proben wurden unverdünnt als Filme aufgetragen, feste Proben wurden in $CH_2Cl_2$ gelöst. Die Absorptionsbanden sind in Wellenzahlen ($cm^{-1}$) angegeben (Messfenster: 4000-650 $cm^{-1}$).

**$^1$H-NMR-Spektren** wurden auf einem Spektrometer des Typs Bruker DPX-300 bei 300.13 MHz gemessen; die chemischen Verschiebungen $\delta$ sind angegeben in ppm relativ zu Tetramethylsilan (TMS), Kopplungskonstanten *J* sind angegeben in Hz. Echte und Pseudo-Kopplungsmuster wurden nicht unterschieden.

Die Viskosität wurde auf einem thermostatisierten Kegel-Platten-Viskosimeter Physica UM (Kegeldurchmesser 20 mm, Kegelwinkel 1 °, Kegelspitze-Platten-Abstand 0.05 mm, Schergeschwindigkeit 10 bis 1000 $s^{-1}$) gemessen.

Der **Amingehalt,** das heisst der totale Gehalt an Aldiminogruppen und freien Aminogruppen in den hergestellten Verbindungen, wurde titrimetrisch bestimmt (mit 0.1N $HClO_4$ in Eisessig, gegen Kristallviolett) und ist stets angegeben in mmol N/g.

Herstellung von Aldehyden

**N-(2,2-Dimethyl-3-oxopropyl)-N-methylacetamid**

In einem Rundkolben mit aufgesetztem Rückflusskühler wurden unter Stickstoffatmosphäre und Rühren 20.0 g (0.17 mol) 2,2-Dimethyl-3-methylamino-propanal vorgelegt. Dazu wurden langsam 30.0 g (0.52 mol) Essigsäureanhydrid zugetropft, die Mischung mit 0.1 g para-Toluolsulfonsäure versetzt und während 1 Std. auf 120 °C erwärmt. Nach Abkühlen auf Raumtemperatur wurde die Reaktionsmischung mit 100 ml Wasser versetzt, mit $Na_2CO_3$ neutralisiert, mehrmals mit Ethylacetat extrahiert, die vereinigte organische Phase über $MgSO_4$ getrocknet und eingeengt. Das so erhaltene braune Öl wurde im Vakuum fraktioniert. Das Produkt destillierte bei einer Kopftemperatur von 93-95 °C und einem Druck von 3 mbar. Ausbeute: 8.2 g (30% d.Th.) farb- und fast geruchlose Flüssigkeit, die beim Stehenlassen kristallisierte.
IR: 3418br, 2971, 2939, 2875, 2815, 1786, 2726, 2711 (CHO), 1718 (C=O Aldehyd), 1624 (C=O Amid), 1487, 1464, 1405, 1364, 1320, 1263, 1202, 1125, 1108, 1086, 1040, 1007, 973, 951, 923, 904, 873, 772, 733.

**N-(2,2-Dimethyl-3-oxopropyl)-pyrrolidin-2-on**

In einem Rundkolben mit aufgesetztem Rückflusskühler wurden unter Stickstoffatmosphäre 40.0 g (0.48 mol) 2-Pyrrolidinon, 39.2 g (0.48 mol) 36%iger wässriger Formaldehyd und 37.3 g (0.52 mol) Isobutyraldehyd vorgelegt unter gutem Rühren mit 10.0 g conc. Salzsäure versetzt, wobei die Mischung heftig aufkochte. Nachdem sich das Kochen beruhigt hatte, wurde die Mischung im Ölbad (100 °C) zum Sieden erhitzt und über Nacht am Kochen gehalten. Die gelbe, klare Reaktionsmischung wurde mit 2N NaOH neutralisiert, zwei Mal mit Ethylacetat extrahiert, die vereinigten organischen Phasen mit Sole gewaschen, über $MgSO_4$ getrocknet und am Rotationsverdampfer vollständig eingeengt. Das erhaltene dunkelgelbe Öl wurde im Vakuum fraktioniert. Das Produkt destillierte bei einer Kopftemperatur von 71 °C und einem Druck von $2 \cdot 10^{-2}$ mbar. Ausbeute: 14.8 g (19% d.Th.) farb- und fast geruchlose Flüssigkeit.
IR: 3429br, 2969, 2961sh, 2930, 2897, 2872, 2837sh, 2786, 2758, 2711 (CHO), 1720 (C=O Aldehyd), 1678 (C=O Amid), 1494, 1462, 1438sh, 1421, 1400, 1380, 1365, 1333, 1314, 1286, 1259, 1225, 1152, 1103, 1062, 1024, 1002,992,975,935,908,890sh,867,772,672.
$^1$H-NMR ($CDCl_3$, 300 K): δ 9.58 (*s*, 1H, CHO), 3.41 (s, 2 H, $NCH_2C(CH_3)_2$), 3.33 (t, *J* = 7.0, 2 H, $\overline{NC(O)CH_2CH_2C}H_2$), 2.36 (*t*, *J* = 8.1, 2 H, $\overline{NC(O)CH_2CH_2CH_2}$), 1.99 (*m*, 2 H, $\overline{NC(O)CH_2CH_2CH_2}$), 1.10 (s, 6 H, $NCH_2C(CH_3)_2$).

**N-(2,2-Dimethyl-3-oxopropyl)-azepan-2-on**

In einem Rundkolben mit aufgesetztem Rückflusskühler wurden unter Stickstoffatmosphäre 80.5 g (0.71 mol) ε-Caprolactam, 59.3 g (0.71 mol) 36%iger wässriger Formaldehyd und 56.4 g (0.78 mol) Isobutyraldehyd vorgelegt unter gutem Rühren mit 13.4 g conc. Salzsäure versetzt (leichte Exothermie). Darauf wurde die Mischung im Ölbad (100 °C) zum Sieden erhitzt und über Nacht am Kochen gehalten. Die gelbliche, klare Reaktionsmischung wurde mit 2N NaOH neutralisiert, zwei Mal mit Ethylacetat extrahiert, die vereinigten organischen Phasen mit Sole gewaschen, über $MgSO_4$ getrocknet und am Rotationsverdampfer vollständig eingeengt. Das erhaltene gelbe Öl wurde im Vakuum fraktioniert. Das Produkt destillierte bei einer Kopftemperatur von 98 °C und einem Druck von $4 \cdot 10^{-2}$ mbar. Ausbeute: 20.7 g (15% d.Th.) farb- und geruchlose Flüssigkeit, die beim Stehenlassen kristallisierte.
IR: 3415br, 2961, 2926, 2855, 2717 (CHO), 1720 (C=O Aldehyd), 1635 (C=O Amid), 1479, 1456, 1443, 1419, 1398, 1365, 1353, 1329, 1310, 1292sh, 1258, 1222, 1197, 1183, 1157, 1137, 1121, 1083, 1060, 1021, 998, 975, 969sh, 954, 944, 923, 909, 890, 872, 839, 815, 771, 735, 723, 703.
$^1$H-NMR ($CDCl_3$, 300 K): δ 9.55 (s, 1H, CHO), 3.47 (s, 2 H, $NCH_2C(CH_3)_2$), 3.36 (*m*, 2 H, $\overline{NC(O)CH_2CH_2CH_2CH_2C}H_2$), 2.52 (*m*, 2 H, $\overline{NC(O)CH_2CH_2CH_2CH_2CH_2}$), 1.67 (*m*, 6 H, $\overline{NC(O)CH_2CH_2CH_2CH_2CH_2}$), 1.09 (*s*, 6 H, $NCH_2C(CH_3)_2$).

**N-(2,2-Dimethyl-3-oxopropyl)-N-methylformamid**

In einem Rundkolben mit aufgesetztem Rückflusskühler wurden unter Stickstoffatmosphäre 59.7 g (1.00 mol) N-Methylformamid, 84.3 g (1.00 mol) 36%iger wässriger Formaldehyd und 78.6 g (1.10 mol) Isobutyraldehyd vorgelegt unter gutem Rühren mit 18.2 g conc. Salzsäure versetzt (leichte Exothermie). Darauf wurde die Mischung im Ölbad (100 °C) zum Sieden erhitzt und über Nacht am Kochen gehalten. Die gelbe, klare Reaktionsmischung wurde auf Raumtemperatur abgekühlt, mit 2N NaOH neutralisiert, zwei Mal mit Ethylacetat extrahiert, die vereinigten organischen Phasen mit Sole gewaschen, über $MgSO_4$ getrocknet und am Rotationsverdampfer vollständig eingeengt. Das erhaltene dunkelgelbe Öl wurde im Vakuum fraktioniert. Das Produkt destillierte bei einer Kopftemperatur von 63 °C und einem Druck von $4 \cdot 10^{-2}$ mbar.

Ausbeute: 20.2 g (14% d.Th.) farblose, leicht stechend riechende Flüssigkeit.
IR: 3412br, 2963, 2931, 2872, 2815sh, 2710 (CHO), 1724 (C=O Aldehyd), 1667 (C=O Amid), 1471, 1444, 1426sh, 1395, 1365, 1302, 1275, 1254, 1181, 1142, 1100, 1071sh, 1047, 990, 973, 953, 914, 883, 858, 772, 747, 710.

<u>Herstellung von Aldiminen</u>

**Beispiel 1: Aldimin *A-1***

In einem Rundkolben wurden unter Stickstoffatmosphäre 2.91 g (18.5 mmol) N-(2,2-Dimethyl-3-oxopropyl)-N-methylacetamid vorgelegt und darin bei Raumtemperatur 1.28 g (15.4 mmol N) 1,6-Hexamethylendiamin-Lösung (70 Gew.-% in Wasser; Amingehalt 12.16 mmol N/g) eingerührt. Danach wurde die Mischung im Ölbad erwärmt und die flüchtigen Bestandteile im Vakuum entfernt (10 mbar, 80 °C). Ausbeute: 3.45 g blassgelbes, fast geruchloses Öl mit einem Amingehalt von 4.74 mmol N/g und einer Viskosität von 630 mPa·s bei 20 °C.
IR: 2959, 2930, 2854, 2827sh, 1644 (C=O, C=N), 1484, 1460, 1430, 1400, 1362, 1312, 1262, 1200, 1181 sh, 1120, 1109, 1086, 1030, 1016, 978, 938, 904,894,860,788,764,730.

**Beispiel 2: Aldimin *A-2***

Unter gleichen Bedingungen wie für Aldimin ***A-1*** beschrieben wurden 10.00 g (59 mmol) N-(2,2-Dimethyl-3-oxopropyl)-pyrrolidin-2-on mit 4.00 g (49 mmol N) 1,6-Hexamethylendiamin (70% in Wasser; Amingehalt 12.16 mmol N/g) umgesetzt. Ausbeute: 11.57 g hellgelbes, fast geruchloses Öl mit einem Amingehalt von 4.13 mmol N/g und einer Viskosität von 2.0 Pa.s bei 20 °C.
IR: 3410br, 2957, 2927, 2854, 2830sh, 1682 (C=O), 1666sh (C=N), 1493, 1461, 1434sh, 1418, 1392, 1363, 1331, 1313, 1284, 1261, 1222, 1150, 1108, 1057, 1023, 993, 944sh, 934, 911, 883, 854, 787, 758, 728, 675, 665.

$^1$H-NMR ($CDCl_3$, 300 K): δ 7.56 (*t, J* = 1.3, 2 H, CH=N), 3.36 (*t, J* = 7.0, 4 H, $NC(O)CH_2CH_2C\mathit{H}_2$ ), 3.35 (*t×d, J* = 7.0/1.3, 4 H, CH=N-$C\mathit{H}_2$), 3.32 (s, 4 H, $NC\mathit{H}_2C(CH_3)_2$), 2.34 (t, *J* = 8.1, 4 H, $NC(O)C\mathit{H}_2CH_2CH_2$ ), 1.97 (*m*, 4 H, $NC(O)CH_2C\mathit{H}_2CH_2$ ), 1.55 (*m*, 4 H, CH=N-$CH_2C\mathit{H}_2$), 1.30 (*m*, 4 H, CH=N-$CH_2CH_2C\mathit{H}_2$), 1.09 (*s*, 12 H, $NCH_2C(C\mathit{H}_3)_2$).

**Beispiel 3: Aldimin *A-3***

Unter gleichen Bedingungen wie für Aldimin ***A-1*** beschrieben wurden 12.67g (64 mmol) N-(2,2-Dimethyl-3-oxopropyl)-azepan-2-on mit 6.46 g (53 mmol N) Polyetherdiamin (Jeffamine® D-230, Huntsman; Amingehalt 8.29 mmol N/g) umgesetzt und im Hochvakuum (4-10$^{-2}$ mbar) bei 120 °C bis zur Gewichtskonstanz eingeengt. Ausbeute: 16.50 g gelber, geruchloser Honig mit einem Amingehalt von 3.18 mmol N/g und einer Viskosität von 23.5 Pa.s bei 20 °C. IR: 3420br, 2964, 2927, 2856, 1645 (C=O und C=N), 1480, 1465sh, 1455, 1444, 1417, 1392, 1367, 1328, 1310, 1291, 1260, 1228, 1196, 1133sh, 1104, 1096sh, 1084sh, 1015, 992, 975, 953, 939, 913, 879, 839, 817, 784, 735, 706.
$^1$H-NMR ($CDCl_3$, 300 K): δ 7.58 (*m*, 2 H, CH=N), 3.6-2.8 (*m*, ca. 19.6 H, $NC\mathit{H}_2C(CH_3)_2$) und $NC(O)CH_2CH_2CH_2CH_2C\mathit{H}_2$ und N/$OCH_2C\mathit{H}(CH_3)$), 2.50 (*m*, 4 H, $NC(O)C\mathit{H}_2CH_2CH_2CH_2CH_2$ ), 1.75-1.55 (*m*, 12 H, $NC(O)CH_2C\mathit{H}_2C\mathit{H}_2C\mathit{H}_2CH_2$ ), 1.15-1.05 (*m*, ca. 23.6 H, $NCH_2C(C\mathit{H}_3)_2$ und N/$OCH_2CH(C\mathit{H}_3)$).

**Beispiel 4: Aldimin *A-4***

Unter gleichen Bedingungen wie für Aldimin ***A-1*** beschrieben wurden 10.45 g (73 mmol) N-(2,2-Dimethyl-3-oxopropyl)-N-methylformamid mit 5.05 g (61 mmol N) 1,6-Hexamethylendiamin-Lösung (70% in Wasser; Amingehalt 12.16 mmol N/g) umgesetzt. Ausbeute: 11.54 gelbliches, leicht stechend riechendes Öl mit einem Amingehalt von 5.03 mmol N/g und einer Viskosität von 1010 mPa·s bei 20 °C.
IR: 3430br, 2955, 2926, 2855, 2829, 1727, 1666 (C=O, C=N), 1465, 1443sh, 1424, 1390, 1362, 1339sh, 1303, 1273, 1254, 1221sh, 1182, 1162sh, 1143, 1093, 1067sh, 1046, 992, 975sh, 953, 932sh, 914, 865, 789, 744, 710, 658.

**Beispiel 5: Aldimin *A-5***

Unter gleichen Bedingungen wie für Aldimin ***A-1*** beschrieben wurden 10.00 g (59 mmol) N-(2,2-Dimethyl-3-oxopropyl)-pyrrolidin-2-on mit 5.07 g (48 mmol N) 2-(2-Aminoethoxy)-ethanol (DGA; Diglycolamine® Agent, Huntsman; Amingehalt 9.46 mmol N/g) umgesetzt. Ausbeute: 14.11 g blassgelbes, fast geruchloses Öl mit einem Amingehalt von 3.39 mmol N/g und einer Viskosität von 630 mPa·s bei 20 °C.
IR: 3400br (OH), 2958, 2925, 2908, 2867, 1680sh (C=O), 1664 (C=N), 1493, 1463, 1438, 1421, 1393, 1363, 1332, 1314, 1287, 1262, 1226, 1123, 1060, 1024sh, 991, 946, 934, 899, 885, 858, 814, 788, 759, 674sh, 669.
$^1$H-NMR ($CDCl_3$, 300 K): δ 7.64 (t, *J* = 1.3, 1 H, CH=N), 3.71 und 3.57 (2×m, 2x4 H, $HOCH_2CH_2OCH_2CH_2N$), 3.40 (t, *J* = 7.0, 2 H, $NC(O)CH_2CH_2CH_2$), 3.31 (s, 2 H, $NCH_2C(CH_3)_2$), 2.35 (t, *J* = 8.1, 2 H, $NC(O)CH_2CH_2CH_2$), 1.98 (*m*, 2 H, $NC(O)CH_2CH_2CH_2$), 1.11 (s, 6 H, $NCH_2C(CH_3)_2$).

Herstellung von härtbaren Zusammensetzungen

**Beispiele 6 bis 10 und Vergleichsbeispiel 11:**

In einem Polypropylenbecher mit Schraubverschluss wurden entweder das Polyurethanpolymer ***P1*** oder das Polyurethanpolymer ***P2,*** deren Herstellung nachfolgend beschrieben wird, mittels eines Zentrifugalmischers (SpeedMixer™ DAC 150, FlackTek Inc.; 1 min. bei 2500 U/min) mit einem Aldimin sowie mit Katalysatoren zu einer homogenen Masse vermischt, die so erhaltene Masse sofort in eine innenlackierte Aluminiumtube abgefüllt und diese luftdicht verschlossen. Die eingesetzten Polyurethanpolymere, Aldimine und Katalysatoren für jedes der Beispiele sind in der Tabelle 1 in Gewichtsteilen aufgeführt.
Das Polyurethanpolymer ***P1*** wurde wie folgt hergestellt:

4000 g Polyoxypropylen-Diol (Acclaim® 4200 N, Bayer; OH-Zahl 28.5 mg KOH/g) und 520 g 4,4'-Methylendiphenyldiisocyanat (4,4'-MDI; Desmodur® 44 MC L, Bayer) wurden nach bekanntem Verfahren bei 80 °C zu einem NCO-terminierten Polyurethanpolymeren mit einem Gehalt an freien Isocyanat-Gruppen von 1.9 Gew.-% umgesetzt.

Das Polyurethanpolymer ***P2*** wurde wie folgt hergestellt:

590 g Polyol Acclaim® 4200 N (Polypropylenoxid-Diol, OH-Zahl 28.5 mg KOH/g; Bayer), 1180 g Polyol Caradol® MD34-02 (Polypropylenoxidpolyethylenoxid-Triol, OH-Zahl 35.0 mg KOH/g; Shell) und 230 g Isophorondiisocyanat (IPDI; Vestanat® IPDI, Degussa) wurden nach bekanntem Verfahren bei 80 °C zu einem NCO-terminierten Polyurethanpolymeren mit einem Gehalt an freien Isocyanat-Gruppen von 2.1 Gew.-% umgesetzt.

Tabelle 1: Zusammensetzung der Beispiele 6 bis 11.

| Beispiel | **6** | **7** | **8** | **9** | **10** | **11 (Vgl.)** |
|---|---|---|---|---|---|---|
| PUR-Polymer ***P1*** | 50.0 | 50.0 | 50.0 | - | - | 50.0 |
| PUR-Polymer ***P2*** | - | - | - | 50.0 | 50.0 | - |
| Aldimin | ***A-1,*** 3.34 | ***A-2,*** 3.84 | ***A-3,*** 4.97 | ***A-4,*** 3.47 | ***A-5,*** 2.58 | - |
| Salicylsäure | 0.05 | 0.05 | 0.05 | 0.05 | 0.05 | - |
| Zinn-Katalysator[a] | 0.05 | 0.05 | 0.05 | 0.05 | 0.05 | - |
| [Aldimin/NCO][b] | 0.70 | 0.70 | 0.70 | 0.70 | 0.35 | - |
| [(Aldimin+HX)/NCO][c] | 0.70 | 0.70 | 0.70 | 0.70 | 0.70 | - |

[a] 5 Gew.-% Dibutylzinndilaurat in Diisodecylphthalat. [b] Verhältnis zwischen der Anzahl Aldiminogruppen und der Anzahl Isocyanatgruppen. [c] Verhältnis der Summe der Anzahl Aldiminogruppen und HX-Gruppen zur Anzahl Isocyanatgruppen.

Die so erhaltenen Zusammensetzungen wurden wie folgt geprüft:

Als Mass für die Lagerstabilität wurde die Veränderung der **Viskosität** während der Lagerung in der Wärme bestimmt. Dazu wurde die Zusammensetzung in der verschlossenen Tube im Ofen bei 60 °C gelagert und die Viskosität bei 20 °C ein erstes Mal nach 4 Stunden (= "Viskosität nach 4h") und ein zweites Mal nach 7 Tagen (= "Viskosität nach 7d") Lagerdauer gemessen. Die Lagerstabilität ergibt sich aus der prozentualen Zunahme des zweiten Viskositätswerts gegenüber dem ersten. Dazu wird die **Viskositätszunahme** in % gemäss folgender Formel berechnet:

$$[(\text{Viskosität nach 7 d} / \text{Viskosität nach 4 h}) - 1] \times 100\%.$$

Zur Messung der **Hautbildungszeit** (Zeit bis zur Klebefreiheit, "tackfree time") wurde ein kleiner Teil der während 4 Stunden bei 60 °C gelagerten Zusammensetzung in einer Schichtdicke von ca. 2 mm auf Pappkarton aufgetragen und im Normklima (23±1 °C, 50±5% relative Luftfeuchtigkeit) die Zeit bestimmt, die es dauerte, bis beim leichten Antippen der Oberfläche der Zusammensetzung mittels einer Pipette aus LDPE erstmals keine Rückstände auf der Pipette mehr zurückblieben.

Zur Bestimmung der mechanischen Eigenschaften wurde mit dem Hauptteil der Zusammensetzung Filme von ca. 3 mm Dicke hergestellt, indem die Zusammensetzung in eine plane PTFE-Form gegossen und während 7 Tagen im Normklima ausgehärtet wurde. Es wurden klare, klebefreie und elastische Polyurethanfilme erhalten, welche vollständig blasenfrei waren. Aus den Filmen wurden Hanteln mit einer Länge von 75 mm, bei einer Steglänge von 30 mm und einer Stegbreite von 4 mm, ausgestanzt und diese gemäss DIN EN 53504 auf **Zugfestigkeit, Bruchdehnung** und **E-Modul** (bei 0.5-5% Dehnung) geprüft (Zuggeschwindigkeit: 200 mm/min).

In qualitativer Weise beurteilt wurde ausserdem die **Blasenbildung** (anhand der Menge Blasen, die während der Aushärtung des Films auftraten) sowie der **Geruch.**

Die Ergebnisse dieser Prüfungen sind in der Tabelle 2 aufgeführt.

Tabelle 2: Eigenschaften der Beispiele 6 bis 11.

| Beispiel | **6** | **7** | **8** | **9** | **10** | **11 (Vgl.)** |
|---|---|---|---|---|---|---|
| Viskosität nach 4h [Pa·s] | 33.1 | 32.0 | 29.5 | 23.1 | 38.7 | 35.3 |
| Viskosität nach 7d [Pa·s] | 38.0 | 34.2 | 33.3 | 30.0 | 58.0 | 38.1 |
| Viskositätszunahme [%] | 15 | 7 | 13 | 30 | 50 | 8 |
| Hautbildungszeit [min.] | 20 | 20 | 45 | 90 | 75 | >480 |
| Zugfestigkeit [MPa] | 3.5 | 2.1 | 1.4 | 1.4 | 1.2 | n.b. |
| Bruchdehnung [%] | 590 | 550 | 720 | 330 | 450 | n.b. |
| E-Modul [MPa] | 9.6 | 8.0 | 1.5 | 1.3 | 1.0 | n.b. |
| Blasenbildung | keine | keine | keine | keine | keine | viele |
| Geruch | gering | gering | kein | gering | gering | kein |
| n.b. steht für "nicht bestimmt" (zuviele Blasen). | | | | | | |

**Patentansprüche**

1. Aldimin der Formel (I)

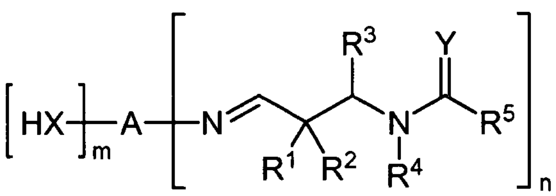

(I)

wobei

Y für O oder S steht;
A entweder

für den (n+m)-wertigen Rest eines Amins nach Entfernung von n primären Aminogruppen und m HX-Gruppen steht,

oder zusammen mit $R^7$ für einen (n+2*m)-wertigen Kohlenwasserstoffrest

mit 3 bis 20 C-Atomen, welcher gegebenenfalls mindestens ein Heteroatom, insbesondere in der Form von Ether-Sauerstoff oder tertiärem Amin-Stickstoff, enthält, steht;

$R^1$ und $R^2$ entweder

unabhängig voneinander jeweils für einen einwertigen Kohlenwasserstoffrest mit 1 bis 12 C-Atomen stehen,

oder zusammen für einen zweiwertigen Kohlenwasserstoffrest mit 4 bis 12 C-Atomen, der Teil eines, gegebenenfalls substituierten, carbocyclischen Rings mit 5 bis 8, bevorzugt 6, C-Atomen ist, stehen;
$R^3$ für ein Wasserstoffatom oder einen Alkyl-, Cycloalkyl-, Arylalkyl- oder Alkoxycarbonylrest mit 1 bis 12 C-Atomen steht;
$R^4$ und $R^5$ entweder

zusammen für einen gegebenenfalls Sauerstoff- oder Schwefelatome aufweisenden zweiwertigen Rest mit 2 bis 10 C-Atomen, der Teil eines, gegebenenfalls substituierten, 5- oder 6- oder 7-gliedrigen Rings ist, stehen,

oder

$R^4$ für einen Alkyl-, Cycloalkyl-, Arylalkyl- oder Acylrest mit 1 bis 10 C-Atomen steht, und
$R^5$ für ein Wasserstoffatom oder für einen einwertigen Rest mit 1 bis 20 C-Atomen ausgewählt aus der Gruppe bestehend aus Alkyl-, Cycloalkyl-, Arylalkyl-, Arylrest, $-OR^{5'}$, $-SR^{5'}$ und $-NR^{5'}R^{5''}$ steht,

wobei $R^{5'}$ und $R^{5''}$ entweder jeweils für einen Kohlenwasserstoffrest oder zusammen für einen Alkylenrest, der Teil eines 5-, 6- oder 7-gliedrigen Rings ist, stehen;

X für O oder S oder $N-R^6$ oder $N-R^7$ steht,

wobei $R^6$
entweder

für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,

oder für einen Substituenten der Formel (II) steht,

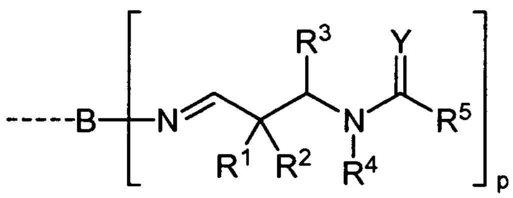

(II)

wobei

p für 0 oder für eine ganze Zahl von 1 bis 10’000 steht, und

B für einen (p+1)-wertigen Kohlenwasserstoffrest, welcher gegebenenfalls Ether-Sauerstoff, tertiären Amin-Stickstoff, Hydroxylgruppen, sekundäre Aminogruppen oder Mercaptogruppen enthält, steht; und

$R^7$ zusammen mit A für einen (n+2*m)-wertigen Kohlenwasserstoffrest mit 3 bis 20 C-Atomen, welcher gegebenenfalls mindestens ein Heteroatom, insbesondere in Form von Ether-Sauerstoff oder tertiärem Amin-Stickstoff, enthält, steht;

n für 1 oder 2 oder 3 oder 4 steht, und
m für 0 oder 1 oder 2 oder 3 oder 4 steht,
mit der Massgabe, dass m+n für 2 oder 3 oder 4 oder 5 steht.

2. Aldimin gemäss Anspruch 1, **dadurch gekennzeichnet, dass** $R^4$ für einen Methyl-, Ethyl-, Propyl-, Isopropyl-, Butyl-, 2-Ethylhexyl-, Cyclohexyl- oder Benzylrest steht,
und dass $R^5$ für Wasserstoff oder für einen Methyl-, Ethyl-, Propyl-, Isopropyl-, Butyl-, 2-Ethylhexyl-, Cyclohexyl-, Benzyl-, Methoxy-, Ethoxy-, Propoxy- oder Isopropoxyrest steht.

3. Aldimin gemäss Anspruch 1, **dadurch gekennzeichnet, dass** $R^4$ und $R^5$ zusammen - unter Einbezug des Stickstoffatoms und der Carbonylgruppe bzw. Thiocarbonylgruppe - einen 2-Pyrrolidon-Ring, einen Pyrrolidin-2,5-dion-Ring, einen Piperidin-2-on-Ring, einen Piperidin-2,6-dion-Ring, einen Azepan-2-on-Ring, einen Oxazolidin-2-on-Ring oder einen Thiazolidin-2-on-Ring, wobei ein solcher Ring gegebenenfalls substituiert ist, bilden.

4. Aldimin gemäss Anspruch 1 bis 3, **dadurch gekennzeichnet, dass** dieses ein Aldimin der Formel (I a) darstellt,

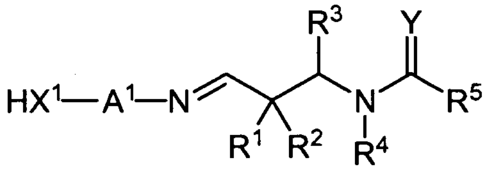
(I a)

wobei

$A^1$ entweder

für einen zweiwertigen Kohlenwasserstoffrest mit 2 bis 20 C-Atomen, welcher gegebenenfalls mindestens ein Heteroatom, insbesondere in der Form von Ether-Sauerstoff oder tertiärem Amin-Stickstoff, enthält, steht,

oder

zusammen mit $R^9$ für einen dreiwertigen Kohlenwasserstoffrest mit 3 bis 20 C-Atomen, welcher gegebenenfalls mindestens ein Heteroatom, insbesondere in der Form von Ether-Sauerstoff oder tertiärem Amin-Stickstoff, enthält, steht;

$X^1$ für O oder S oder N-$R^8$ oder N-$R^9$ steht,

wobei $R^8$
entweder

für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,

oder für einen Substituenten der Formel (II a) steht,

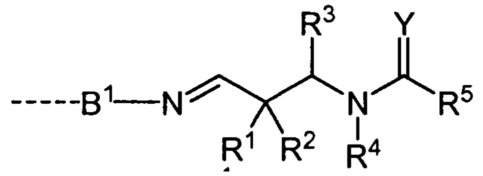

(II a)

wobei $B^1$ für einen zweiwertigen, gegebenenfalls Ether-Sauerstoff oder tertiären Amin-Stickstoff aufweisenden, Kohlenwasserstoffrest mit 2 bis 12 C-Atomen steht; und

$R^9$ zusammen mit $A^1$ für einen dreiwertigen Kohlenwasserstoffrest mit 3 bis 20 C-Atomen, welcher gegebenenfalls mindestens ein Heteroatom, insbesondere in der Form von Ether-Sauerstoff oder tertiärem Amin-Stickstoff, enthält, steht;

mit der Massgabe, dass $A^1$ keinen aktiven Wasserstoff aufweist.

**5.** Aldimin gemäss Anspruch 4, **dadurch gekennzeichnet, dass** es aus der Umsetzung von mindestens einem Amin **B1** ausgewählt aus der Gruppe bestehend aus N-Methyl-1,2-ethandiamin, N-Ethyl-1,2-ethandiamin, N-Cyclohexyl-1,2-ethandiamin, N-Methyl-1,3-propandiamin, N-Ethyl-1,3-propandiamin, N-Butyl-1,3-propandiamin, N-Cyclohexyl-1,3-propandiamin, 4-Aminomethyl-piperidin, 3-(4-Aminobutyl)-piperidin, Diethylentriamin (DETA), Dipropylentriamin (DPTA), Bis-hexamethylentriamin (BHMT), Fettdiamine, insbesondere N-Cocoalkyl-1,3-propandiamin, N-Oleyl-1,3-propandiamin, N-Soyaalkyl-1,3-propandiamin und N-Talgalkyl-1,3-propandiamin; 5-Amino-1-pentanol, 6-Amino-1-hexanol, 4-(2-Aminoethyl)-2-hydroxyethylbenzol, 3-Aminomethyl-3,5,5-trimethyl-cyclohexanol, 2-(2-Aminoethoxy)-ethanol, Triethylenglykol-monoamin, 3-(2-Hydroxyethoxy)-propylamin, 3-(2-(2-Hydroxyethoxy)-ethoxy)-propylamin und 3-(6-Hydroxyhexyloxy)-propylamin mit mindestens einem Aldehyd **ALD** der Formel (IV) erhalten wird

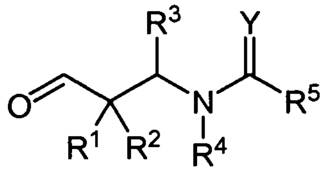

(IV).

**6.** Aldimin gemäss Anspruch 1 bis 3, **dadurch gekennzeichnet, dass** dieses ein Aldimin der Formel (I b) darstellt,

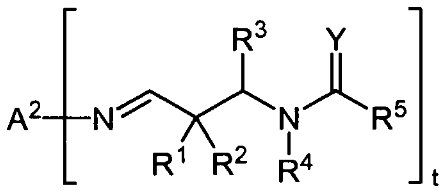

(I b)

wobei

t für 2 oder 3 steht;
$A^2$ für den Rest eines Amins **B2** nach Entfernung von t primären Aminogruppen steht,
mit der Massgabe, dass das Aldimin der Formel (I b) keinen aktiven Wasserstoff aufweist.

**7.** Aldimin gemäss Anspruch 6, **dadurch gekennzeichnet, dass** das Amin **B2** ausgewählt ist aus der Gruppe bestehend aus 1,6-Hexamethylendiamin, 1,5-Diamino-2-methylpentan (MPMD), 1,3-Pentandiamin (DAMP), 1-Amino-3-aminomethyl-3,5,5-trimethylcyclohexan (= Isophorondiamin oder IPDA), 2,2,4- und 2,4,4-Trimethylhexamethylendiamin (TMD), 1,3-Xylylendiamin, 1,3-Bis-(aminomethyl)cyclohexan, Bis-(4-aminocyclohexyl)-methan, Bis-(4-amino-3-methylcyclohexyl)-methan, 3(4),8(9)-Bis-(aminomethyl)-tricyclo[5.2.1.$0^{2,6}$]decan, 1,2-, 1,3- und 1,4-Diaminocyclohexan, 1,4-Diamino-2,2,6-trimethylcyclohexan, 3,6-Dioxaoctan-1,8-diamin, 4,7-Dioxadecan-1,10-diamin, 4-Aminomethyl-1,8-octandiamin, Polyoxyalkylen-Polyaminen mit zwei oder drei Aminogruppen, 1,3- und 1,4-Phenylendiamin, 2,4- und 2,6-Toluylendiamin, 4,4'-, 2,4'- und 2,2'-Diaminodiphenylmethan, 3,3'-Dichlor-4,4'-diaminodiphenylmethan und Mischungen der genannten Polyamine.

**8.** Aldimin gemäss einem der vorhergehenden Ansprüche, erhalten aus der Umsetzung von mindestens einem Amin **B1** oder **B2** mit mindestens einem Aldehyd **ALD** der Formel (IV)

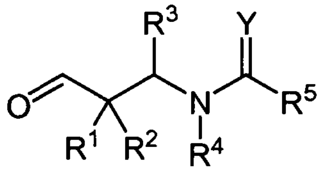 (IV),

ausgewählt aus der Gruppe bestehend aus N-(2,2-Dimethyl-3-oxopropyl)-N-methylformamid, N-(2,2-Dimethyl-3-oxopropyl)-N-methylacetamid, N-(2,2-Dimethyl-3-oxopropyl)-N-butylacetamid, N-(2,2-Dimethyl-3-oxopropyl)-N-(2-ethylhexyl)acetamid, N-(2,2-Dimethyl-3-oxopropyl)-N-benzylacetamid, N-(2,2-Dimethyl-3-oxopropyl)-N-methylbutyramid, N-(2,2-Dimethyl-3-oxopropyl)-N-methyl-(2-ethylcapronamid), N-(2,2-Dimethyl-3-oxopropyl)-N-methylbenzamid, O-Ethyl-N-(2,2-dimethyl-3-oxopropyl)-N-methylcarbamat, N-(2,2-Dimethyl-3-oxopropyl)-pyrrolidin-2-on, N-(2,2-Dimethyl-3-oxopropyl)-piperidin-2-on, N-(2,2-Dimethyl-3-oxopropyl)-azepan-2-on, N-(2,2-Dimethyl-3-oxopropyl)-oxazolidin-2-on, N-(2,2-Dimethyl-3-oxopropyl)-thiazolidin-2-on, N-(2,2-Dimethyl-3-oxopropyl)-pyrrolidin-2,5-dion und N-(2,2-Dimethyl-3-oxopropyl)-phthalimid.

**9.** Aldimin gemäss einem der vorhergehenden Ansprüche, **dadurch gekennzeichnet, dass** es ausgehend von einem Zwischenprodukt **ZW2** der Formel (X) erhalten wird,

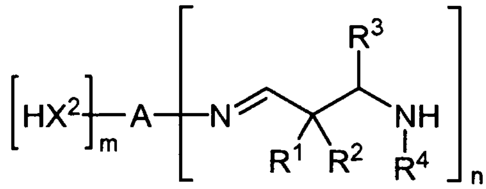 (X)

wobei $X^2$ für O oder S steht.

**10.** Additionsprodukt **AV** der Formel (XII), erhalten aus der Umsetzung von mindestens einem Polyisocyanat **P** mit mindestens einem Aldimin der Formel (I a) gemäss einem der Ansprüche 4 oder 5 oder 8,

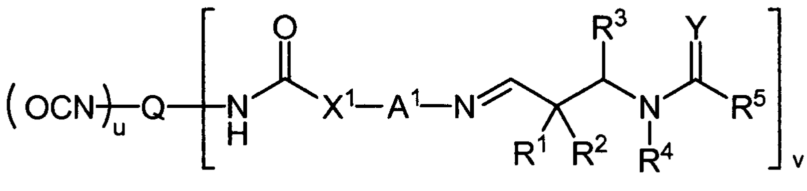 (XII)

wobei Q für den Rest eines (u+v) Isocyanatgruppen aufweisenden Polyisocyanates **P** nach Entfernung aller Isocyanatgruppen steht;
u für 0 oder 1 oder 2 oder 3 steht,
v für 1 oder 2 oder 3 oder 4 steht,
mit der Massgabe, dass (u+v) für 2 oder 3 oder 4 steht.

**11.** Einkomponentige feuchtigkeitshärtende Zusammensetzung, enthaltend

a) mindestens ein Polyisocyanat **P** und
b) mindestens ein Aldimin der Formel (I b) gemäss einem der Ansprüche 6 bis 8 und/oder mindestens ein

Additionsprodukt **AV** der Formel (XII) gemäss Anspruch 10.

**12.** Einkomponentige feuchtigkeitshärtende Zusammensetzung gemäss Anspruch 11, **dadurch gekennzeichnet, dass** das Polyisocyanat **P** ein aromatische Isocyanatgruppen aufweisendes Polyurethanpolymer **PUP,** welches erhältlich ist durch die Umsetzung von mindestens einem Polyol mit mindestens einem aromatischen monomeren Diisocyanat, darstellt.

**13.** Verfahren zum Verkleben von einem Substrat **S1** mit einem Substrat **S2** umfassend die Schritte

i) Applikation einer einkomponentigen feuchtigkeitshärtenden Zusammensetzung gemäss Anspruch 11 oder 12 auf ein Substrat **S1;**
ii) Kontaktieren der applizierten Zusammensetzung mit einem Substrat **S2** innerhalb der Offenzeit der Zusammensetzung;

oder

i') Applikation einer einkomponentigen feuchtigkeitshärtenden Zusammensetzung gemäss Anspruch 11 oder 12 auf ein Substrat **S1** und auf ein Substrat **S2;**
ii') Kontaktieren der applizierten Zusammensetzung miteinander innerhalb der Offenzeit der Zusammensetzung;

wobei das Substrat **S2** aus dem gleichen oder einem unterschiedlichen Material wie das Substrat **S1** besteht.

**14.** Ausgehärtete Zusammensetzung erhalten aus der Reaktion einer einkomponentigen feuchtigkeitshärtenden Zusammensetzung gemäss Anspruch 11 oder 12 mit Feuchtigkeit, insbesondere in Form von Luftfeuchtigkeit.

**15.** Verwendung eines Aldimins der Formel (I) gemäss einem der Ansprüche 1 bis 9 oder einem Additionsprodukt **AV** der Formel (XII) gemäss Anspruch 10 in Klebstoffen, Dichtstoffen, Beschichtungen oder Bodenbelägen.

## Claims

**1.** Aldimine of Formula (I)

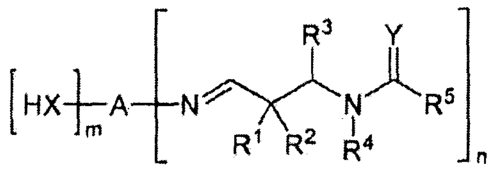

(I)

whereby

Y stands for O or S;
A either

stands for the (n+m)-value radical of an amine after removal of n primary amino groups and m HX groups,

or together with $R^7$ stands for an (n+2*m)-value hydrocarbon radical with 3 to 20 C atoms, which optionally contains at least one heteroatom, in particular in the form of ether-oxygen or tertiary amine-nitrogen;
$R^1$ and $R^2$ either

independently of one another in each case stand for a monovalent hydrocarbon radical with 1 to 12 C atoms,

or together stand for a divalent hydrocarbon radical with 4 to 12 C atoms, which is part of an optionally substituted, carbocyclic ring with 5 to 8, preferably 6, C atoms;
$R^3$ stands for a hydrogen atom or an alkyl, cycloalkyl, arylalkyl or alkoxycarbonyl radical with 1 to 12 C atoms;
$R^4$ and $R^5$ either

together stand for a divalent radical with 2 to 10 C atoms that optionally has oxygen or sulfur atoms and that is part of an optionally substituted, 5- or 6- or 7-membered ring,

or
$R^4$ stands for an alkyl, cycloalkyl, arylalkyl or acyl radical with I to 10 C atoms, and
$R^5$ stands for a hydrogen atom or for a monovalent radical with I to 20 C atoms selected from the group that consists of an alkyl, cycloalkyl, arylalkyl, or aryl radical, $-OR^{5'}$, $-SR^{5'}$ and $-NR^{5'}R^{5'}$,

whereby $R^{5'}$ and $R^{5'}$ either in each case stand for a hydrocarbon radical or together stand for an alkylene radical, which is part of a 5-, 6- or 7-membered ring;

X stands for O or S or $N-R^6$ or $N-R^7$,

whereby $R^6$
either

stands for a monovalent hydrocarbon radical with 1 to 20 C atoms, which has optionally at least one carboxylic acid ester, nitrile, nitro, phosphonic acid ester, sulfone or sulfonic acid ester group,

or stands for a substituent of Formula (II),

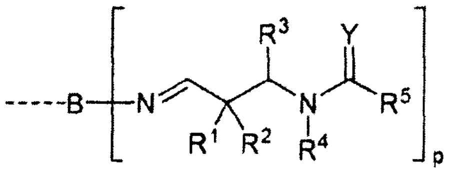

(II)

whereby

p stands for 0 or for an integer from I to 10,000, and
B stands for a (p+1)-value hydrocarbon radical, which optionally contains ether-oxygen, tertiary amine-nitrogen, hydroxyl groups, secondary amino groups or mercapto groups; and

$R^7$ together with A stands for an (n+2*m)-value hydrocarbon radical with 3 to 20 C atoms, which optionally contains at least one heteroatom, in particular in the form of ether-oxygen or tertiary amine-nitrogen;

n stands for 1 or 2 or 3 or 4, and
m stands for 0 or 1 or 2 or 3 or 4,
provided that m+n stands for 2 or 3 or 4 or 5.

**2.** Aldimine according to Claim 1, **characterized in that** $R^4$ stands for a methyl, ethyl, propyl, isopropyl, butyl, 2-ethylhexyl, cyclohexyl or benzyl radical,
and **in that** $R^5$ stands for hydrogen or for a methyl, ethyl, propyl, isopropyl, butyl, 2-ethylhexyl, cyclohexyl, benzyl, methoxy, ethoxy, propoxy or isopropoxy radical.

**3.** Aldimine according to Claim I, wherein $R^4$ and $R^5$ together- with inclusion of the nitrogen atom and the carbonyl or thiocarbonyl group - form a 2-pyrrolidone ring, a pyrrolidine-2,5-dione ring, a piperidin-2-one ring, a piperidine-2,6-dione ring, an azepan-2-one ring, an oxazolidin-2-one ring or a thiazolidin-2-one ring, whereby such a ring is optionally substituted.

**4.** Aldimine according to Claims I to 3, wherein the latter represents an aldimine of Formula (Ia)

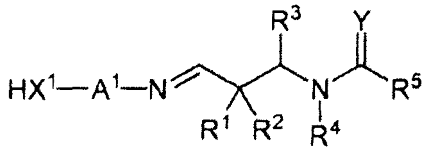

(I a)

whereby

$A^1$ either

stands for a divalent hydrocarbon radical with 2 to 20 C atoms, which optionally contains at least one heteroatom, especially in the form of ether-oxygen or tertiary amine-nitrogen,

or

together with $R^9$ stands for a trivalent hydrocarbon radical with 3 to 20 C atoms, which optionally contains at least one heteroatom, in particular in the form of ether-oxygen or tertiary amine-nitrogen,

$X^1$ stands for O or S or N-$R^8$ or N-$R^9$,

whereby $R^8$
either

stands for a monovalent hydrocarbon radical with I to 20 C atoms, which optionally has at least one carboxylic acid ester, nitrile, nitro, phosphonic acid ester, sulfone or sulfonic acid ester group,

or stands for a substituent of Formula (II a),

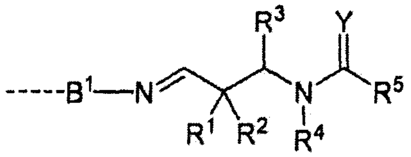

(II a)

whereby $B^1$ stands for a divalent hydrocarbon radical with 2 to 12 C atoms that optionally has ether-oxygen or tertiary amine-nitragen; and

$R^9$ together with $A^1$ stands for a trivalent hydrocarbon radical with 3 to 20 C atoms, which optionally contains at least one heteroatom, in particular in the form of ether-oxygen or tertiary amine-nitrogen;
provided that $A^1$ does not have any active hydrogen.

**5.** Aldimine according to Claim 4, wherein it is obtained from the reaction of at least one amine **B1** that is selected from the group that consists of N-methyl-1,2-ethanediamine, N-ethyl-1,2-ethanediamine, N-cyclohexyl-1,2-ethanediamine, N-methyl-1,3-propanediamine, N-ethyl-1,3-propanediamine, N-butyl-1,3-propanediamine, N-cyctohexyl-1,3-propanediamine, 4-aminomethyl-piperidine, 3-(4-aminobutyl)-piperidine, diethylenetriamine (DETA), dipropylenetriamine (DPTA), bishexamethylenetriamine (BHMT), fatty diamines, in particular N-cocoalkyl-1,3-propanediamine, N-oleyl-1,3-propanediamine, N-soya alkyl-1,3-propanediamine and N-taliowalkyl-1,3-propanediamine; 5-amino-1-pentanol, 6-amino-1-hexanol, 4-(2-aminoethyl)-2-hydroxyethylbenzene, 3-aminomethyl-3,5,5-trimethylcyclohexanol, 2-(2-aminoethoxy)-ethanol, triethylene glycol monoamine, 3-(2-hydroxyethoxy)-propylamine, 3-(2-(2-hydroxyetboxy)-ethoxy)-propylamine and 3-(6-hydroxyhexyloxy)-propylamine with at least one aldehyde **ALD** of Formula (IV).

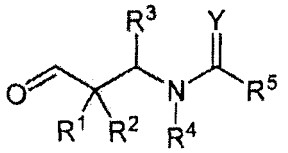

(IV).

6. Aldimine according to Claims 1 to 3, wherein the latter represents an aldimine of Formula (Ib),

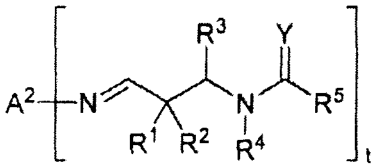

(I b)

whereby

t stands for 2 or 3;
$A^2$ stands for the radical of an amine **B2** after removal of t primary amino groups,
provided that the aldimine of Farmu(a (Ib) does not have any active hydrogen.

7. Aldimine according to Claim 6, wherein the amine **B2** is selected from the group that consists of 1,6-hexamethylenediamine, 1,5-diamino-2-methylpentane (MPMD), 1,3-pentanediamine (DAMP), 1-amino-3-aminomethyl-3,5,5-trimethylcyclohexane (= isophorone diamine or IPDA), 2,2,4- and 2,4,4-trimethylhexamethylenediamine (TMD), 1,3-xylylenediamine, 1,3-bis-(aminomethyl)cyclohexane, bis-(4-aminocyclohexyl)-methane, bis-(4-amino-3-methytcyclohexyl)-methane, 3(4),8(9)-bis-(aminomethyl)-tricyclo[5.2.1.0$^{2,6}$]decane, 1,2-, 1,3- and 1,4-diaminocyclohexane, 1,4-diamino-2,2,6-trimethylcyclohexane, 3,6-dioxaoctane-1,8-diamine, 4,7-dioxadecane-1,10-diamine, 4-aminamethyl-1,8-octanediamine, polyoxyalkylene polyamines with two or three amino groups, 1,3- and 1,4-phenylenediamine, 2,4- and 2,6-toluylenediamine, 4,4'-, 2,4'- and 2,2'-diaminodiphenylmethane, 3,3'-dichloro-4,4'-diaminodiphenylmethane and mixtures of the above-mentioned polyamines.

8. Aldimine according to one of the preceding claims, obtained from the reaction by at least one amine **B1** or **B2** with at least one aldehyde **ALD** of Formula (IV)

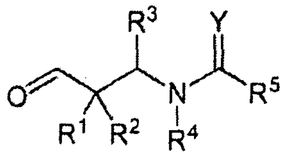

(IV),

selected from the group that consists of N-(2,2-dimethyl-3-oxopropyl)-N-methylformamide, N-(2,2-dimethyl-3-oxopropyl)-N-methylacetamide, N-(2,2-dimethyl-3-oxopropyl)-N-butylacetamide, N-(2,2-dimethyl-3-oxapropyl)-N-(2-ethylhexyl)acetamide, N-(2,2-dimethyl-3-oxopropyl)-N-benzylacetamide, N-(2,2-dimethyl-3-oxopropyl)-N-methylbutyramide, N-(2,2-dimethyl-3-oxopropyl)-N-methyl-(2-ethylcapronamide), N-(2,2-dimethyl-3-oxopropyl)-N-methylbenzamide, O-ethyl-N-(2,2-dimethyl-3-oxopropyl)-N-methylcarbamate, N-(2,2-dimethyl-3-oxopropyl)-pyrrolidin-2-one, N-(2,2-dimethyl-3-oxopropyl)-piperidin-2-one, N-(2,2-dimethyl-3-oxopropyl)-azepan-2-one, N-(2,2-dimethyl-3-oxopropyl)-oxazolidin-2-one, N-(2,2-dimethyl-3-oxopropyl)-thiazolidin-2-one, N-(2,2-dimethyl-3-oxopropyl)-pyrrolidine-2,5-dione, and N-(2,2-dimethyl-3-oxopropyl)-phthalimide.

9. Aldimine according to one of the preceding claims, wherein it is obtained starting from an intermediate product **ZW2** of Formula (X),

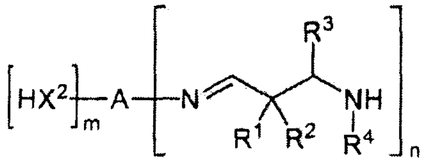

(X)

whereby $X^2$ stands for O or S.

**10.** Addition product AV of Formula (XII), obtained from the reaction of at least one polyisocyanate P with at least one aldimine of Formula (I a) according to one of Claims 4 or 5 or 8,

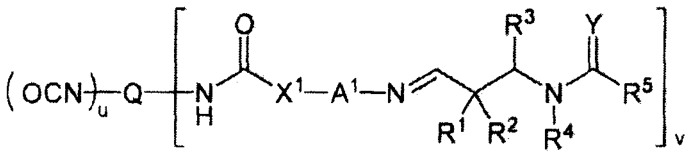

(XII)

whereby Q stands for the radical of a (u+v) polyisocyanate P that has isocyanate groups after removal of all isocyanate groups;
u stands for 0 or 1 or 2 or 3,
v stands for 1 or 2 or 3 or 4,
provided that (u+v) stands for 2 or 3 or 4.

**11.** Single-component, moisture-curing composition, containing

a) At least one polyisocyanate **P**, and
b) At least one aldimine of Formula (I b) according to one of Claims 6 to **8** and/or at least one addition product **AV** of Formula (XII) according to Claim 10.

**12.** Single-component, moisture-curing composition according to Claim 11, wherein the polyisocyanate P represents a polyurethane polymer **PUP** that has aromatic isocyanate groups and that is available by the reaction of at least one polyol with at least one aromatic monomeric diisocyanate.

**13.** Method for adhesive bonding a substrate **S1** to a substrate **S2** that comprises the steps:

i) Application of a single-component, moisture-curing composition according to Claim 11 or 12 on a substrate **S1**;
ii) Ensuring contact of the applied composition with a substrate **S2** within the open time of the composition;

or

i') Application of a single-component, moisture-curing composition according to Claim 11 or 12 on a substrate **S1** and on a substrate **S2**;
ii') Ensuring contact of the applied composition together within the open time of the composition;

whereby the substrate **S2** consists of the same material or a different material such as the substrate **S1**.

**14.** Cured composition obtained from the reaction of a single-component. moisture-curing composition according to Claim 11 or 12 with moisture, in particular in the form of atmospheric humidity.

**15.** Use of an aldimine of Formula (I) according to one of Claims I to 9 or an addition product **AV** of Formula (XII) according to Claim 10 in adhesives, sealants, coatings or floor coverings.

## Revendications

1. Aldimine de formule (I)

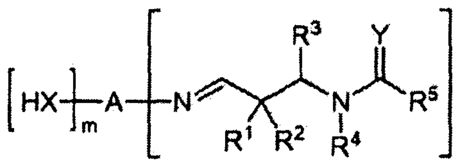

(I)

où

Y représente O ou S ;
A représente soit le radical organique (n + m)-valent d'une amine après l'élimination de n groupes amino primaires et de m groupes HX,
soit, conjointement avec $R^7$, un radical hydrocarboné (n + 2 * m)-valent comprenant 3 à 20 atomes de carbone, qui contient le cas échéant au moins un hétéroatome, en particulier sous forme d'oxygène de fonction éther ou d'azote de fonction amine tertiaire ;
$R^1$ et $R^2$ représentent
soit, indépendamment l'un de l'autre, à chaque fois un radical hydrocarboné monovalent comprenant 1 à 12 atomes de carbone,
soit conjointement un radical hydrocarboné divalent comprenant 4 à 12 atomes de carbone, qui fait partie d'un cycle carbocyclique, le cas échéant substitué, comprenant 5 à 8, de préférence 6, atomes de carbone ;
$R^3$ représente un atome d'hydrogène ou un groupe alkyle, cycloalkyle, arylalkyle ou alcoxycarbonyle comprenant 1 à 12 atomes de carbone;
$R^4$ et $R^5$ représentent
soit conjointement un radical divalent présentant le cas échéant des atomes d'oxygène ou de soufre comprenant 2 à 10 atomes de carbone, qui fait partie d'un cycle de 5, 6 ou 7 chaînons, le cas échéant substitué, soit
$R^4$ représente un radical alkyle, cycloalkyle, aralkyle ou aryle comprenant 1 à 10 atomes de carbone, et
$R^5$ représente une atome d'hydrogène ou un radical monovalent comprenant 1 à 20 atomes de carbone, choisi dans le groupe constitué par un radical alkyle, cycloalkyle, arylalkyle, aryle, $-OR^{5'}$, $-SR^{5'}$ et $-NR^{5'}R^{5''}$,

où $R^{5'}$ et $R^{5'}$ représentent soit chacun un radical hydrocarboné, soit conjointement un radical alkylène, qui fait partie d'un cycle de 5, 6 ou 7 chaînons;
X représente O ou S ou N-$R^6$ ou N-$R^7$, où
$R^6$ représente
soit un radical hydrocarboné monovalent comprenant 1 à 20 atomes de carbone, qui présente le cas échéant au moins un groupe ester d'acide carboxylique, nitrile, nitro, ester d'acide phosphonique, sulfone ou ester d'acide sulfonique,
soit un substituant de formule (II),

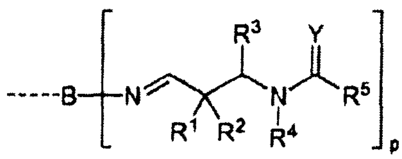

(II)

où

p vaut 0 ou un nombre entier de 1 à 10 000, et
B représente un radical hydrocarboné (p + 1)-valent, qui contient le cas échéant de l'oxygène de fonction éther, de l'azote de fonction amine tertiaire, des groupes hydroxyle, des groupes amino secondaires ou des groupes mercapto ; et

$R^7$ représente, conjointement avec A, un radical hydrocarboné (n + 2 * m)-valent comprenant 3 à 20 atomes de carbone, qui contient le cas échéant au moins un hétéroatome, en particulier sous forme d'oxygène de

fonction éther ou d'azote de fonction amine tertiaire ;

n vaut 1 ou 2 ou 3 ou 4, et
m vaut 0 ou 1 ou 2 ou 3 ou 4,
à condition que m + n vaut 2 ou 3 ou 4 ou 5.

**2.** Aldimine selon la revendication 1, **caractérisée en ce que** $R^4$ représente un radical méthyle, éthyle, propyle, isopropyle, butyle, 2-éthylhexyle, cyclohexyle ou benzoyle, et
**en ce que** $R^5$ représente hydrogène ou un radical méthyle, éthyle, propyle, isopropyle, butyle, 2-éthylhexyle, cyclohexyle, benzyle, méthoxy, éthoxy, propoxy ou isopropoxy.

**3.** Aldimine selon la revendication 1, **caractérisée en ce que** $R^4$ et $R^5$ forment conjointement - en intégrait l'atome d'azote et le groupe carbonyle ou, selon le cas, le groupe thiocarbonyle - un cycle 2-pyrrolidone, un cycle pyrrolidine-2,5-dione, un cycle pipéridin-2-one, un cycle pipéridine-2,6-dione, un cycle azépan-2-one, un cycle oxazolidin-2-one ou un cycle thiazolidin-2-one, un tel cycle étant le cas échéant substitué.

**4.** Aldimine selon la revendication 1 à 3, **caractérisée en ce qu'**il s'agit d'une aldimine de formule (Ia),

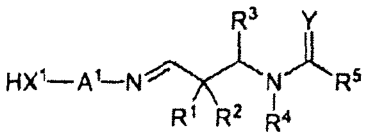

(I a)

où

$A^1$ représente
soit un radical hydrocarboné divalent comprenant 2 à 20 atomes de carbone, qui contient le cas échéant au moins un hétéroatome, en particulier sous forme d'oxygène de fonction éther ou d'azote de fonction amine tertiaire,
soit, conjointement avec $R^9$, un radical hydrocarboné trivalent comprenant 3 à 20 atomes de carbone, qui contient le cas échéant au moins un hétéroatome, en particulier sous forme d'oxygène de fonction éther ou d'azote de fonction amine tertiaire ;

$X^1$ représente 0 ou S ou N-$R^8$ ou N-$R^9$, où
$R^8$ représente
soit un radical hydrocarboné monovalent comprenant 1 à 20 atomes de carbone, qui présente le cas échéant au moins un groupe ester d'acide carboxylique, nitrile, nitro, ester d'acide phosphonique, sulfone ou ester d'acide sulfonique,
soit un substituant de formule (IIa),

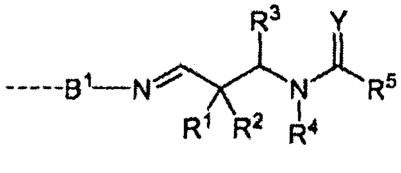

(II a)

où $B^1$ représente un radical hydrocarboné divalent, présentant le cas échéant un oxygène de fonction éther ou un azote de fonction amine tertiaire, comprenant 2 à 12 atomes de carboné ; et

$R^5$ représente, conjointement avec $A^1$, un radical hydrocarboné trivalent comprenant 3 à 20 atomes de carbone, qui contient le cas échéant au moins un hétéroatome, en particulier sous forme d'oxygène de fonction éther ou d'azote de fonction amine tertiaire ;

à condition que $A^1$ ne présente pas d'hydrogène actif.

**5.** Aldimine selon à revendication 4, **caractérisée en ce qu'**elle est obtenue par transformation d'au moins une amine **B1** choisie dans le groupe constitué par la N-méthyl-1,2-éthanediamine, la N-éthyl-1,2-éthanediamine, la a N-cyclohexyl-1,2-éthanediamine, la N-méthyl-1,3-propanediamine, la N-éthyl-1,3-propanediamine, la N-butyl-1,3-propanediamine, la N-cyclohexyl-1,3-propanediamine, la 4-aminométhylpipéridine, la 3-(4-aminobutyl)-pipéridine, la diéthylènetriamine (DETA), la dipropylènetriamine (DPTA), la bishexaméthylènetriamine (BHMT), les diamines grasses, en particulier la N-coco-alkyl-1,3-propanediamine, la N-oléyl-1,3-propanediamine, la N-soja-alkyl-1,3-propanediamine et la N-suif-alkyl-1,3-propanediamine ; le 5-amino-1-pentanol, le 6-amino-1-hexanol, le 4-(2-aminoéthyl)-2-hydroxyéthylbenzène, le 3-aminométhyl-3,5,5-triméthylcyclohexanol, le 2-(2-aminoéthoxy)-méthanol, la triéthylèneglycolmonoamine, la 3-(2-hydroxyéthoxy)-propylamine, la 3-(2-(2-hydroxyéthoxy)-éthoxy)-propylamine et la 3-(6-hydroxyhexyloxy)-propylamine comprenant au moins un aldéhyde **ALD** de formule (IV)

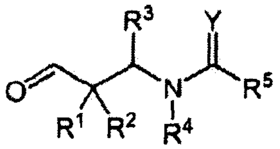

(IV).

**6.** Aldimine selon la revendication 1 à 3, **caractérisée en ce qu'**il s'agit d'une aldimine de formule (Ib),

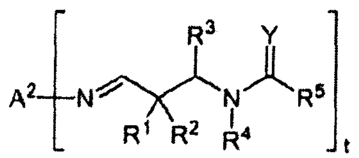

(I b)

où

t vaut 2 ou 3 ;
$A^2$ représente le radical d'une amine **B2** apurés l'élimination de t groupes amino primaires,

à condition que l'aldimine de formule (Ib) ne présente pas d'hydrogène actif.

**7.** Aldimine selon la revendication 6, **caractérisée en ce que** l'amine **B2** est choisie dans le groupe constitué par la 1,6-hexaméthylènediamine, le 1,5-diamino-2-méthylpentane (MPMD), la 1,3-pentanediamine (DAMP), le 1-amino-3-aminométhyl-3,5,5-triméthylcyclohexane (= isophoronediamine ou IPDA), la 2,2,4-triméthylhexaméthylènediamine et la 2,4,4-triméthylhexaméthylènediamine (TMD), la 1,3-xylylènediamine, le 1,3-bis-(aminonméthyl)cyclohexane, le bis-(4-aminocyclohexyl)-méthane, le bis-(4-amino-3-méthylcyclohexyl)-méthane, le 3(4),8(9)-bris-(aminométhyl)-tricyclo[5.2.1.$0^{2,6}$]décane, le 1,2-diaminocyclohexane, le 1,3-diaminocyclohexane et le 1,4-diaminocyclohexane, le 1,4-diamino-2,2,6-triméthylcyclohexane, la 3,6-dioxaoctane-1,8-diamine, la 4,7-dioxadécane-1,10-diamine, la 4-aminométhyl-1,8-octanediamine, les polyoxyalkylène-polyamines comprenant deux ou trois groupes amino, la 1,3-phénylènediamine et la 1,4-phénylènediamine, la 2,4-toluyènediamine et la 2,6-toluylènediamine, le 4,4'-diaminodiphénylméthane, le 2,4'-diaminodiphénylméthane et le 2,2'-diaminodiphénylméthane, le 3,3'-dichlaro-4,4'-diaminodiphénylméthane et les mélanges des polyamines mentionnées.

**8.** Aldimine selon l'une quelconque des revendications précédentes, obtenue à partir de la transformation d'au moins une amine **B1** ou **B2** avec au moins un aldéhyde ALD de formule (IV)

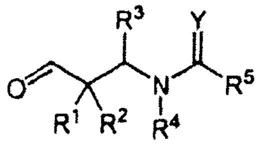

(IV),

choisi dans le groupe constitué par le K-(2,2-diméthyl-3-oxopropyl)-N-methylformamide, le N-(2,2-diméthyl-3-oxopropyl)-N-méthylacétamide, le N-(2,2-diméthyl-3-oxopropyl)-N-butylacétamide, le N-(2,2-diméthyl-3-oxopropyl)-N-(2-éthylhexyl)acétamide, le N-(2,2-diméthyl-3-oxopropyl)-N-benzylacétamide, le N-(2,2-diméthyl-3-oxopropyl)-N-méthylbutyramide, le N-(2,2-diméthyl-3-oxopropyl)-N-méthyl-(2-éthylcapronamide), le N-(2,2-diméthyl-3-oxopropyl)-N-méthylbenzamide, l'O-éthyl-N-(2,2-diméthyl-3-oxopropyl)-N-méthylcarbamate, la N-(2,2-diméthyl-3-oxopropyl)-pyrrolidin-2-one, la N-(2,2-diméthyl-3-oxopropyl)-pipéridin-2-one, la -N-(2,2-diméthyl-3-oxopropyl)-azépan-2-one, la N-(2,2-diméthyl-3-oxopropyl)-oxazolidin-2-one, la N-(2,2-diméthyl-3-oxopropyl)-thiazolidin-2-one, la N-(2,2-diméthyl-3-oxopropyl)-pyrrolidine-2,5-dione et le N-(2,2-diméthyl-3-oxopropyl)-phtalimide.

**9.** Aldimine selon l'une quelconque des revendications précédentes, **caractérisée en ce qu'**elle est obtenue à partir d'un produit intermédiaire **ZW2** de formule (X),

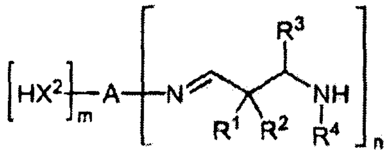

(X)

où $X^2$ représente O ou S.

**10.** Produit d'addition **AV** de formule (XII), obtenu à partir de la transformation d'au moins un polyisocyanate **P** avec au moins une aldimine de formule (Ia) selon l'une quelconque des revendications 4 ou 5 ou 8,

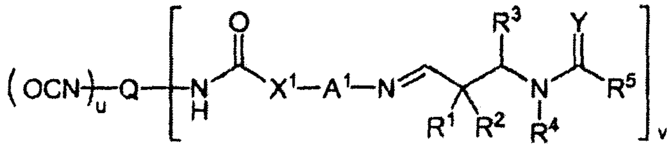

(XII)

où Q représente le radical d'un polyisocyanate **P** présentant (u + v) groupes isocyanate, après élimination de tous les groupes isocyanate ;

u vaut 0 ou 1 ou 2 ou 3,
v vaut 1 ou 2 ou 3 ou 4,

à condition que (u + v) vaut 2 ou 3 ou 4.

**11.** Composition durcissant sous l'effet de l'humidité, à un composant, contentant

a) au moins un polyisocyanate **P** et
b) au moins une Aldimine de formule (Ib) selon l'une quelconque des revendications 6 à 8 et/ou au moins un produit d'addition **AV** de formule (XII) selon la revendication 10.

**12.** Composition durcissait sous l'effet de l'humidité, à un composant selon la revendication 11, **caractérisée en ce que** le Polyisocyanate **P** est un polymère de polyuréthane **PUP** présentant des groupes isocyanate aromatiques, qui peut être obtenu par transformation d'au moins un polyol avec au moins un diisocyanate monomère aromatique.

**13.** Procédé pour le collage d'un substrat **S1** avec un substrat **S2** comprenant les étapes

i) application d'une composition durcissant sous l'effet de l'humidité, à un composant selon la revendication 11 ou 12 sur un substrat **S1** ;
ii) mise en contact de la composition appliquée avec un substrat **S2** pendant le temps ouvert de la composition ; ou
i') application d'une composition durcissant sous l'effet de l'humidité, à un composant selon la revendication 11 ou 12 sur un substrat S1 et sur un substrat **S2** ;
ii') mise en contact de la composition appliquée l'une avec l'autre pendant le temps ouvert de la composition ;

le substrat **S2** étant constitué par le même matériau que le substrat **S1** ou par un matériau différent du substrat **S1**.

**14.** Composition durcie e obtenue par la réaction d'une composition durcissant sous l'effet de l'humidité, à un composant selon la revendication 11 ou 12 avec de l'humidité, en particulier sous forme de l'humidité de l'air.

**15.** Utilisation d'une aldimine de formule (I) selon l'une quelconque des revendications 1 à 9 ou d'un produit d'addition **AV** de formule (XII) selon la revendication 10 dans des adhésifs, des substances d'étanchéité, des revêtements ou des recouvrements de sol.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2004013088 A1
- WO 2007036571 A1